# EUROPEAN PATENT APPLICATION

(11) **EP 1 961 745 A1**
(43) Date of publication of application: **27.08.2008**
(21) Application number: 06834425.8
(22) Date of filing: 11.12.2006
(51) Int. Cl.: C07D 235/18, A61K 31/4184, A61K 31/437, A61K 45/00, A61P 1/00, A61P 15/00, A61P 25/00, A61P 25/14, A61P 25/18, A61P 25/22, A61P 25/24, A61P 25/30, A61P 43/00, C07D 471/04

(54) **BICYCLIC HETEROCYCLIC COMPOUND**

(30) Priority: 12.12.2005 JP 2005357758
(71) Applicant: ONO PHARMACEUTICAL CO., LTD., Osaka-shi, Osaka 541-8526 (JP)
(72) Inventor: NAKAI, Hisao, Mishima-gun Osaka 618-8585 (JP); SAITO, Tetsuji, Mishima-gun Osaka 618-8585 (JP); KAGAMIISHI, Yoshifumi, Mishima-gun Osaka 618-8585 (JP)
(74) Representative: Henkel, Feiler & Hänzel
(86) International application number: PCT/JP2006/324670
(87) International publication number: WO 2007/069565

(57) **Abstract**

A compound represented by the formula (I), a salt thereof, an N-oxide thereof, a solvate thereof, or a prodrug thereof: 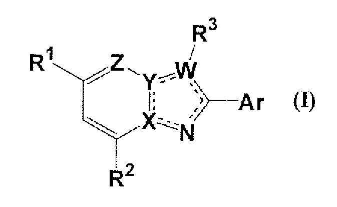 wherein X, Y, and W each independently represent a carbon atom or a nitrogen atom;
Z represents CH or a nitrogen atom;
R¹ represents (1) C3-10 branched alkyl which may be substituted or (2) -(CH₂)ₘ-NR⁴R⁵; R² and R³ each independently represent (1) a hydrogen atom, (2) C1-4 alkyl which may be substituted with a halogen atom, hydroxy which may be protected, amino which may be protected, or carboxyl which may be protected, (3) C2-4 alkenyl, (4) C2-4 alkynyl, (5) nitrile, (6) COOR⁶, (7) CONR⁷R⁸, (8) COR¹⁰¹, (9) S(O)ₙR¹⁰², or (10) a halogen atom, in which R⁶ represents a hydrogen atom or C1-4 alkyl, R⁷ and R⁸ each independently represent a hydrogen atom or C1-4 alkyl, R¹⁰¹ represents a hydrogen atom or C1-4 alkyl,
R¹⁰² represents C1-4 alkyl, n represents 1 or 2; and Ar represents an aromatic ring which may be substituted, is useful as a pharmacologically active ingredient having a CRF antagonist action in preventing and/or treating neuropsychiatric diseases, diseases of peripheral organs or the like.

## Description

### Technical Field

The present invention relates to a novel bicyclic heterocyclic ring compound or a salt thereof, and a pharmaceutical containing as an active ingredient the same. More specifically, the present invention relates to a novel bicyclic heterocyclic ring compound represented by the formula (I), a salt thereof, an N-oxide thereof, a solvate thereof, or a prodrug thereof, and a pharmaceutical containing as an active ingredient the same: 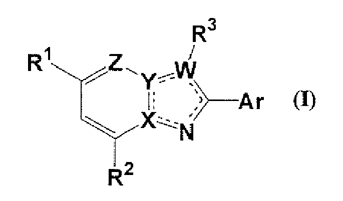 wherein all symbols represent the same meanings as described hereinafter.

### Background Art

Corticotropin Releasing Factor (CRF) is a peptide including 41 amino acid isolated from ovine hypothalamic in 1981. It was suggested that CRF was released from hypothalamic and controlled a secretion of adrenocorticotropic hormone (ACTH) from hypophysis [Science, 218, 377-379 (1982)].

ACTH, which is released by a stimulation of CRF, stimulates a secretion of cortisol from adrenal cortex, and relates to a systemic action for reproduction, growth, gastrointestinal function, inflammation, immune system, nervous system, etc. Consequently, CRF is believed to play a role as a regulator of these functions. In view of those things, an involvement of CRF with a neuropsychiatric disease or a disease of peripheral organs has received attention.

On the other hand, the depression patients and the anxiety disorder patients increase, and the number also of depression patients with the slight illness increases recently. Moreover, an aged patient is commanding a majority in the depression patient. Under these circumstances, from the earliness of the appearance of the effect and in view of the side effect, neuropsychiatric disease treatment which can be easily used is requested more and more.

Currently, for the treatment of neuropsychiatric diseases, for example, tricyclic antidepressants, tetracyclic antidepressants, monoamine oxidase inhibitors, serotonin and noradrenaline reuptake inhibitors (SNRI), selective serotonin reuptake inhibitors (SSRI), etc. as antidepressant are used. However, the therapeutic gain is not enough; it will take a long time by the time the effect appears; drowsiness, a dryness of the mouth, constipation, difficulty feelings in micturition, etc. are seen as a side effect. As an antianxiety agent, such as benzodiazepine anxiolytic, thienodiazepine anxiolytic, non-benzodiazepine anxiolytic etc. are used. However, the therapeutic gain is not also enough; decrease in mental movement function and decrease in concentration and attention power, drowsiness, stagger, dizziness, headache, amnesia, etc. are seen as a side effect.

As the bicyclic heterocyclic compound, WO 2005/026126 describes that a compound represented by the formula (A) has a CRF antagonistic action: 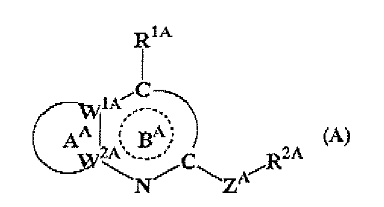 wherein A^{A} ring represents a 5- or 6-msmbered monocycle which may be substituted with 1 to 3 substituent(s);
B^{A} ring represents a 5-7 membered monocyclic unsaturated heterocycle which may additionally contain or substituted with one or two heteroatoms selected from a nitrogen atom, an oxygen atom, and/or a sulfur atom which may be oxidized other than a nitrogen atom, W^{1A}, and W^{2A};
W^{1A} and W^{2A} each independently represent a carbon atom or a nitrogen atom;
Z^{A} represents -NR^{3A}-, an oxygen atom, a sulfur atom which may be oxidized, or -CR^{4A}R^{5A}-;
R^{1A} represents (i) optionally substituted, C1-15 alkyl, C2-15 alkenyl, or C2-15 alkynyl, (ii) an amino which may be protected, (iii) hydroxyl which may be protected, (iv) mercapto which may be protected, (v) -S(O)_{nA}R^{6A}, (vi) -COR^{7A}, or (vii) a cyclic group which may be substituted; and
R^{2A} represents an unsaturated cyclic group which may be substituted.

In addition, WO 2005/044793 describes that the compound represented by the formula (B) has a CRF antagonistic action; 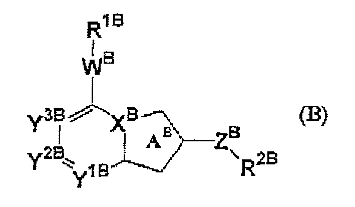 wherein A^{B} ring represents a 5-membered ring represented by the following formula: 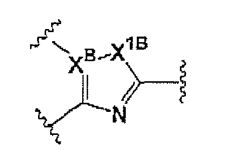 in the ring, X^{B} represents a carbon atom and X^{1B} represents an oxygen atom, a sulfur atom, or -NR^{5B}-, or: 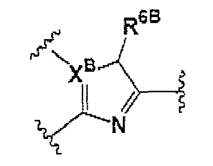 in the ring, X^{B} represents a nitrogen atom and R^{6B} represents a hydrogen atom, optionally substituted hydrocarbon, or an acyl;
R^{1B} represents (1) amino substituted by two substituents or (2) optionally substituted cyclic amino;
R^{2B} represents optionally substituted alkyl, optionally substituted cycloalkyl, optionally substituted allyl, or the like;
Y^{1B}, Y^{2B}, and Y^{3B} each independently represent optionally substituted methine or a nitrogen atom;
W^{B} represents a bond, -(CH₂)_{nB}-, or -(CH₂)_{mB}-CO-;
Z^{B} represents a bond, -CO-, an oxygen atom, or a sulfur atom.

In addition, WO 1999/040091 describes that the compound represented by the formula (C) has a neuropeptide Y antagonistic action and a CRF antagonistic action; 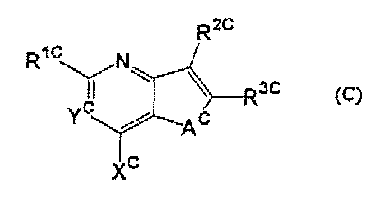 wherein Y^{C} represents N or C(R^{6C});
A^{C} represents NH, NR^{4C}, or CR^{4C}R7^{C};
R^{1C} represents a hydrogen atom, a halogen atom, OH, NO₂, NHOH, CF₃, OCF₃, C1-8 alkyl, C3-10 cycloalkyl, or the like;
R^{2C} represents a hydrogen atom, a halogen atom, OH, NO₂, CF₃, OCF₃, C1-8 alkyl, C3-10 cycloalkyl, or the like;
R^{3C} represents C3-10 cycloalkyl, C1-8 alkyl, C1-8 alkyl-OH, - D^{C}' (allyl), -D^{C}' (heteroallyl), and the like;
X^{C} represents C1-8 alkyl, C3-10 cycloalkyl, -Z^{C}(allyl), -
Z^{C}(heteroallyl), or the like;
D^{C}' represents -(C1-8 alkyl)_{kC}-;
k^{C} represents 0 or 1;
Z^{C} represents D^{C}(NR^{5C})_{KC} or the like; and
D^{C} represents -(CH₂)_{mC}(C3-10 cycloalkyl)_{KC}(CH₂)_{mC}-,

In addition, WO 2002/066477 describes that the compound represented by the formula (D) and having an imidazopyridine skeleton has an antagonist action of gonadotropin releasing hormone; 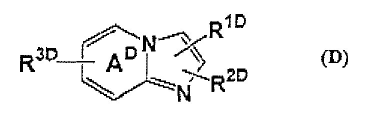 wherein R^{1D} and R^{2D} are each independently selected from a hydrogen atom and a group bonded through a carbon atom, a nitrogen atom, an oxygen atom or a sulfur atom, R^{3D} is selected from a group bonded through a heteroatom, an optionally substituted C1-20 hydrocarbon group, and an optionally substituted C1-6 alkyl group; and ring A is optionally further substituted.
[Patent Document 1] WO 2005/026126
[Patent Document 2] WO 2005/044793
[Patent Document 3] WO 1999/040091
[Patent Document 4] WO 2002/066477

### Disclosure of the Invention

### Problem to be solved by the Invention

It is desired to obtain an agent which is easily handled and has potent prevention and/or treatment effects in the prevention and/or treatment of neuropsychiatric diseases, diseases of peripheral organs or the like.

### Means for solving the Problem

The inventors of the present invention studied intensively in order to solve the above problems, and as a result, found that the object can be achieved by a bicyclic heterocyclic compound.

That is, the present invention relates to:
[1] A compound represented by formula (I), a salt thereof, an N-oxide thereof, a solvate thereof, or a prodrug thereof: 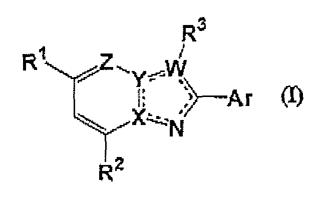 wherein X, Y, and W each independently represent a carbon atom or a nitrogen atom;
   Z represents CH or a nitrogen atom;
   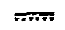 represents a single or double bond, which does not represents a double bond successively;
   R¹ represents (1) C3-10 branched alkyl which may be substituted or (2) -(CH₂)ₘ-NR⁴R⁵, in which R⁴ and R⁵ each independently represent C1-6 alkyl which may be substituted, or R⁴ represents a hydrogen atom, and R⁵ represents C3-6 branched alkyl which may be substituted, m represents 0 or an integer of 1-3;
   R² and R³ each independently represent (1) a hydrogen atom, (2) C1-4 alkyl which may be substituted with a halogen atom, hydroxy which may be protected, amino which may be protected, or carboxyl which may be protected, (3) C2-4 alkenyl, (4) C2-4 alkynyl, (5) nitrile, (6) COOR⁶, (7) CONR⁷R⁸, (8) COR¹⁰¹, (9) S(O)ₙR¹⁰², or (10) a halogen atom, in which R⁶ represents a hydrogen atom or C1-4 alkyl, R⁷ and R⁸ each independently represent a hydrogen atom or C1-4 alkyl, R¹⁰¹ represents a hydrogen atom or C1-4 alkyl, R¹⁰² represents C1-4 alkyl, n represents 1 or 2; and
   Ar represents an aromatic ring which may be substituted;
[2] The compound according to [1], a salt thereof, an N-oxide thereof, a solvate thereof, or a prodrug thereof, wherein 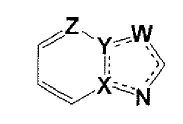 is 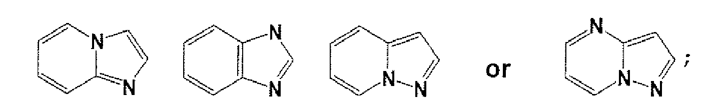
[3] The compound according to [1], a salt thereof, an N-oxide thereof, a solvate thereof, or a prodrug thereof, wherein R¹ is C3-10 branched alkyl which may be substituted;
[4] The compound according to [1], a salt thereof, an N-oxide thereof, a solvate thereof, or a prodrug thereof, wherein Ar has 1-3 substituent(s), and is a 5-12 membered monocyclic or bicyclic aromatic ring which may contain 1-4 hetero atoms selected from a nitrogen atom, oxygen atom and/or sulfur atom which may be oxidized;
[5] The compound according to [4], a salt thereof, an N-oxide thereof, a solvate thereof, or a prodrug thereof, wherein Ar is a benzene having 1-3 substituent(s);
[6] The compound according to [1], a salt thereof, an N-oxide thereof, a solvate thereof, or a prodrug thereof, wherein the formula (I) is formula (I-A-1): 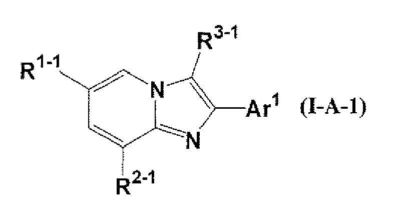 wherein R¹⁻¹ represents unsubstituted C3-10 branched alkyl,
   R²⁻¹ represents a hydrogen atom, unsubstituted C1-4 alkyl or nitrile,
   R³⁻¹ represents a hydrogen atom, C1-4 alkyl which may be substituted with a hydroxy which may be protected, nitrile, COOR⁶, CONR⁷R⁸, COR¹⁰¹, or S (O)ₙR¹⁰², in which all symbols represent the same meanings as described in [1],
   Ar¹ represents a benzene having 1-3 substituent(s);
[7] The compound according to [1], a salt thereof, an N-oxide thereof, a solvate thereof, or a prodrug thereof, wherein the formula (I) is formula (I-B-1): 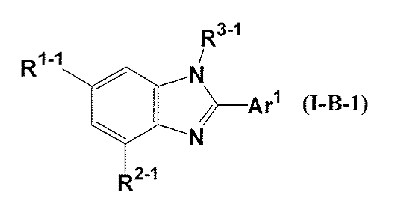 in which all symbols represent the same meanings as described in [6];
[8] The compound according to [1], a salt thereof, an N-oxide thereof, a solvate thereof, or a prodrug thereof, wherein the formula (I) is formula (I-C-1): 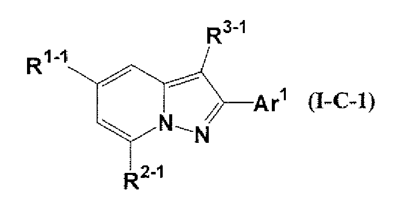 in which all symbols represent the same meanings as described in [6] ;
[9] The compound according to [6], a salt thereof, an N-oxide thereof, a solvate thereof, or a prodrug thereof, wherein the formula (I-A-1) is formula (I-A-1-1): 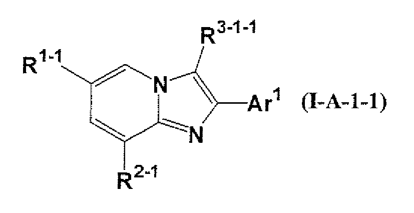 wherein R³⁻¹⁻¹ represents unsubstituted C1-4 alkyl, or CONR⁷R⁸, in which all symbols represent the same meanings as described in [1] and the other symbols represent the same meaning as described in [6];
[10] The compound according to [1], a salt thereof, an N-oxide thereof, a solvate thereof, or a prodrug thereof, wherein the compound represented by the formula (I) is
   (1) 2-(2-chloro-4-methoxyphenyl)-6-(1-ethylpropyl)-3,8-dimethylimidazo[1,2-a]pyridine,
   (2) 3-ethyl-6-(1-ethylpropyl)-2-(4-methoxy-2-methylphenyl)-8-methylimidazo[1,2-a]pyridine,
   (3) 2-(2-chloro-4-methoxyphenyl)-3-ethyl-6-(1-ethylpropyl)-8-methylimidazo[2,2-a]pyridine,
   (4) ethyl 2-(2-chloro-4-methoxyphenyl)-6-(1-ethylpropyl)-8-methylimidazo[1,2-a]pyridine-3-carboxylate,
   (5) methyl 2-(2-chloro-4-methoxyphenyl)-6-(1-ethylpropyl)-8-methylimidazo[1,2-a]pyridine-3-carboxylate,
   (6) 2-(2-chloro-4-methoxyphenyl)-6-(1-ethylpropyl)-8-methylimidazo[1,2-a]pyridine-3-carboxamide,
   (7) 2-(2-chloro-4-methoxyphenyl)-6-(1-ethylpropyl)-N,8-dimethylimidazo[1,2-a]pyridine-3-carboxamide,
   (8) 2-(2-chloro-4-methoxyphenyl)-6-(1-ethylpropyl)-1,4-dimethyl-1H-benzimidazole, or
   (9)2-(2-chloro-4-methoxyphenyl)-5-(1-ethylpropyl)-7-methylpyrazolo[1,5-a]pyridine-3-carboxamide;
[11] The compound according to [6], a salt thereof, an N-oxide thereof, a solvate thereof, or a prodrug thereof, wherein the compound represented by the formula (I-A-1) is
   (1) 6-(1-ethylpropyl)-2-(4-methoxy-2-methylphenyl)-8-methylimidazo[1,2-a]pyridine-3-carboxamide,
   (2)6-(1-ethylpropyl)-8-methyl-2-(2,4,5-trimethylphenyl)imidazo[1,2-a]pyridine-3-carboxamide,
   (3) 2-(2-ethyl-4-methoxyphenyl)-6-(1-ethylpropyl)-8-methylimidazo[1,2-a]pyridine-3-carboxamide,
   (4) 2-(4-ethoxy-2-ethylphenyl)-6-(1-ethylpropyl)-8-methylimidazo[1,2-a]pyridine-3-carboxamide,
   (5) 2-(2-chloro-4-methoxy-5-methylphenyl)-6-(1-ethylpropyl)-8-methylimidazo[1,2-a]pyridine-3-carboxamide,
   (6) 1-[2-(2-chloro-4-methoxyphenyl)-6-(1-ethylpropyl)-8-methylimidazo[1,2-a]pyridin-3-yl]ethanone,
   (7) 2-(4-ethoxy-2-methylphenyl)-6-(1-ethylpropyl)-8-methylimidazo[1,2-a]pyridine-3-carboxamide, or
   (8) 6-(1-ethylpropyl)-2-(4-methoxy-2,5-dimethylphenyl)-8-methylimidazo[1,2-a]pyridine-3-carboxamide;
[12] A pharmaceutical composition comprising as an active ingredient the compound represented by the formula (I) described in [1], a salt thereof, an N-oxide thereof, a solvate thereof, or a prodrug thereof;
[13] The pharmaceutical composition according to [12], which is a CRF antagonist;
[14] The pharmaceutical composition according to [13], which is an agent for preventing and/or treating CRF mediated diseases;
[15] The pharmaceutical composition according to [14], wherein the CRF mediated diseases are neuropsychiatric diseases or digestive diseases;
[16] The pharmaceutical composition according to [15], wherein the neuropsychiatric diseases or the digestive diseases are mood disorder, anxiety disorder, adjustment disorder, stress-related disorder, eating disorder, symptom caused by psychotropic substance or dependency thereon, organic mental disorder, schizophrenic disorder, attention-deficit hyperactivity disorder, irritable bowel syndrome, or gastrointestinal disorder caused by stress;
[17] The pharmaceutical composition according to [16], wherein the mood disorders are depression, bipolar disorder, indefinite complaint, premenstrual dysphoric disorder, postpartum mood disorder, or perimenopausal or menopausal dysphoric disorder, and the anxiety disorders are generalized anxiety disorder, panic disorder, obsessive compulsive disorder, social anxiety disorder, or phobic disorder;
[18] A pharmaceutical composition comprising the compound represented by the formula (I) described in [1], a salt thereof, an N-oxide thereof, a solvate thereof, or a prodrug thereof in combination with at least one kind selected from a tricyclic antidepressant, a tetracyclic, antidepressant, a monoamine oxidase inhibitor, a serotonin and noradrenaline reuptake inhibitor, a selective serotonin reuptake inhibitor, a serotonin reuptake inhibitor, a psychostimulant, an antianxiety agent, an antipsychotic agent, a mitochondrial benzodiazepine receptor ligand, a neurokinin 1 antagonist, a gastrointestinal promotility agent, a histamine H₂ receptor antagonist, a proton pump inhibitor, a 5-HT₃ antagonist, a 5-HT₄ agonist, an anticholinergic agent, an antidiarrheal drug, a laxative, and an autonomic modulating agent;
[19] A method of preventing and/or treating CRF mediated diseases, comprising administering to a mammal an effective amount of the compound represented by the formula (I) described in [1], a salt thereof, an N-oxide thereof, a solvate thereof, or a prodrug thereof; and
[20] A use of the compound represented by the formula (I) described in [1], a salt thereof, an N-oxide thereof, a solvate thereof, or a prodrug thereof for manufacturing an agent for preventing and/or treating CRF mediated diseases. Effect of the Present Invention

The bicyclic heterocyclic ring compound of the present invention strongly binds to a CRF receptor to exhibit a strong antagonist action.

### Best Modes for carrying out the Invention

In the present invention, in the ring represent by the following formula, 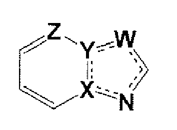 X, Y, and W each independently represent a carbon atom or a nitrogen atom, and Z represents CH or a nitrogen atom. In the case where the Z is a nitrogen atom, it is preferred that X represents a nitrogen atom. Further, it is preferred that the number of nitrogen atoms in the ring be within three including one which has already been described. In particular, 2 or 3 in total number are preferred. Specifically, the following ring is given:

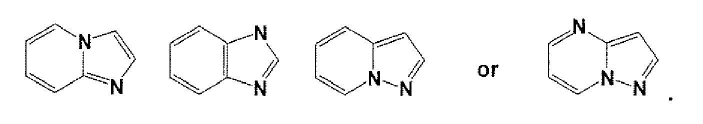

In the present invention, "C3-10 branched alkyl which may be substituted" is "C3-10 branched alkyl substituted with a substituent(s)" or "unsubstituted C3-10 branched alkyl".

In the present invention, "C3-10 branched alkyl" in "C3-10 branched alkyl which may be substituted", "C3-10 branched alkyl substituted with a substituent(s)" and "unsubstituted C3-10 branched alkyl" includes branched propyl, butyl, pentyl, hexyl, heptyl, octyl, nonyl and decyl.

Specifically, isopropyl, isobutyl, sec-butyl, tert-butyl, 1-methylbutyl, 1-ethylpropyl, 1,1-dimethylpropyl, 1,2-dimethylpropyl, 2-methylbutyl, 3-methylbutyl, 2,2-dimethylpropyl, 1-methylpentyl, 1-ethylbutyl, 2-ethylbutyl, 1-ethyl-1-methylpropyl, 1-ethyl-2-methylpropyl, 1,1-dimethylbutyl, 1,2-dimethylbutyl, 1,3-dimethylbutyl, 2-methylpentyl, 3-methylpentyl, 4-methylpentyl, 2,3-dimethylbutyl, 1-methylhexyl, 1-ethylpentyl, 2-ethylpentyl, 1-propylbutyl, 2-methyl-3-hexyl, 1,2-dimethylpentyl, 1,3-dimethylpentyl, 1,4-dimethylpentyl, 1-ethyl-1-methylbutyl, 1-methyl-2-ethylbutyl, 1-ethyl-2-methylbutyl, 1-ethyl-3-methylbutyl, 1,1-dimethylpentyl, 1,1,3-trimethylbutyl, 1,1-diethylpropyl, 2-methylhexyl, 3-methylhexyl, 4-methylhexyl, 5-methylhexyl, 3-ethylpentyl, 1-methylheptyl, 2-methylheptyl, 3-methylheptyl, 4-methylheptyl, 5-methylheptyl, 6-methylheptyl, 1-ethylhexyl, 2-ethylhexyl, 3-ethylhexyl, 1-propylpentyl, 2-propylpentyl, 1,5-dimethylhexyl, 1-ethyl-4-methylpentyl, 1-propyl-3-methylbutyl, 1,1-dimethylhexyl, 1-ethyl-1-methylpentyl, 1,1-diethylbutyl, 1-methyloctyl, 2-methyloctyl, 7-methyloctyl, 1-ethylheptyl, 2-ethylheptyl, 1-propylhexyl, 2-propylhexyl, 3-ethylheptyl, 3-propylhexyl, 1-butylpentyl, 1,6-dimethylheptyl, 1-ethyl-5-methylhexyl, 1-propyl-4-methylpentyl, 1-butyl-3-methylbutyl, 1,1-dimethylheptyl, 1-ethyl-1-methylhexyl, 1,1-diethylpentyl, 1-ethyl-1-propylbutyl, 1-methylnonyl, 2-methylnonyl, 1-ethyloctyl, 2-ethyloctyl, 3-ethyloctyl, 1-propylheptyl, 2-propylheptyl, 3-propylheptyl, 1-butylhexyl, 2-butylhexyl, 3-butylhexyl 1,8-dimethyloctyl, 1-ethyl-6-methylheptyl, 1-propyl-5-methylhexyl, 1-butyl-4-methylpentyl, 1,1-dimethyloctyl, 1-ethyl-1-methylheptyl, 1-ethyl-1-propylpentyl, 1,1-dipropylbutyl, and the like are given.

In the present invention, as the "substituent(s)" in "the C3-10 branched alkyl substituted with a substituent(s)" include hydroxyl, C1-4 alkoxy, a halogen atom, -CF₃, -OCF₃, C3-6 cycloalkyl, -O-(C3-6 cycloalkyl), C5-6 monocyclic unsaturated carbocyclic ring, -O-(C5-6 monocyclic unsaturated carbocyclic ring), a 3-6 membered monocyclic heterocyclic ring, -O-(3-6 membered monocyclic heterocyclic ring). Those substituents may be arbitrary substituted in the C3-10 branched alkyl at substitutable positions, but 1 to 4 substitutable positions are preferred.

In the present invention, "C1-4 alkyl which may be substituted with a halogen atom, hydroxy which may be protected, amino which may be protected, or carboxyl which may be protected" represents "C1-4 alkyl which may be substituted with a halogen atom, hydroxy which may be protected, amino which may be protected, or carboxyl which may be protected", or "unsubstituted C1-4 alkyl".

In the present invention, the "C1-4 alkyl" in the "C1-4 alkyl which may be substituted with a halogen atom, hydroxy which may be protected, amino which may be protected, or carboxyl which may be protected", "C1-4 alkyl substituted with a halogen atom, hydroxy which may be protected, amino which may be protected, or carboxyl which may be protected" and "unsubstituted C1-4 alkyl" represents straight or branched C1-4 alkyl, and includes methyl, ethyl, n-propyl, isopropyl, n-butyl, isobutyl, sec-butyl, and tert-butyl.

In the present invention, the "halogen atom", "hydroxy which may be protected", "amino which may be protected" and "carboxyl which may be protected" in the "C1-4 alkyl which may be substituted with a halogen atom, hydroxy which may be protected, amino which may be protected, or carboxyl which may be protected" and "C1-4 alkyl substituted with a halogen atom, hydroxy which may be protected, amino which may be protected, or carboxyl which may be protected" each represent the same meanings with "a halogen atom", "hydroxy which may be protected", "amino which may be protected" and "carboxyl which may be protected" described later.

In the present invention, "C1-6 alkyl which may be substituted" represents "C1-6 alkyl substituted with a substituent(s)" or "unsubstituted C1-6 alkyl".

In the present invention, the "C1-6 alkyl" in the "C1-6 alkyl which may be substituted", "C1-6 alkyl substituted with a substituent(s)" and "unsubstituted C1-6 alkyl" represents straight or branched C1-6 alkyl, and includes methyl, ethyl, n-propyl, isopropyl, n-butyl, isobutyl, sec-butyl, tert-butyl, pentyl, hexyl, and isomers thereof.

In the present invention, as the "substituent(s)" in the "C1-6 alkyl substituted with a substituent(s)", hydroxyl, C1-4 alkoxy, a halogen atom, -CF₃, -OCF₃, C3-6 cycloalkyl, -O-(C3-6 cycloalkyl), C5-6 monocyclic unsaturated carbocyclic ring, -O-(C5-6 monocyclic unsaturated carbocyclic ring), a 3-6 membered monocyclic heterocyclic ring, and -O-(3-6 membered monocyclic heterocyclic ring) are given. Those substituents may be arbitrary substituted in the C1-6 alkyl at substitutable positions, but 1 to 4 substitutable positions are preferred.

In the present invention, "C3-6 branched alkyl which may be substituted" represents "C3-6 branched alkyl substituted with a substituent(s)" or "unsubstituted C3-6 alkyl."

In the present invention, the "C3-6 branched alkyl" in the "C3-6 branched alkyl which may be substituted", "C3-6 branched alkyl substituted with a substituent(s)" and "unsubstituted C3-6 alkyl" represents branched propyl, butyl, pentyl, and hexyl. For example, isopropyl, sec-butyl, tert-butyl, 1-methylbutyl, 1-ethylpropyl, 1,1-dimethylpropyl, 1,2-dimethylpropyl, 1-methylpentyl, 1-ethylbutyl, 1-ethyl-1-methylpropyl, 1-ethyl-2-methylpropyl, 1,1-dimethylbutyl, 1,2-dimethylbutyl, and 1,3-dimethylbutyl are given.

In the present invention, as the "substituent(s)" in the "C3-6 branched alkyl substituted with a substituent(s)" include hydroxyl, C1-4 alkoxy, a halogen atom, -CF₃, -OCF₃, C3-6 cycloalkyl, -O-(C3-6 cycloalkyl), C5-6 monocyclic unsaturated carbocyclic ring, -O-(C5-6 monocyclic unsaturated carbocyclic ring), a 3-6 membered monocyclic heterocyclic ring, and -O-(3-6 membered monocyclic heterocyclic ring). Those substituents may be arbitrary substituted in the C3-6 branched alkyl at substitutable positions, but 1 to 4 substitutable positions are preferred.

In the present invention, the "halogen atom" includes fluorine, chlorine, bromine and iodine. In the present invention, the "C1-4 alkyl substituted with a halogen atom" is C1-4 alkyl substituted with 1-5 atoms selected from fluorine, chlorine, bromine, and iodine, preferably, C1-4 alkyl substituted with 1-3 same atoms selected from fluorine, chlorine, bromine, and iodine.

In the present invention, "C2-4 alkenyl" includes ethenyl, 1-propenyl, 2-propenyl, 1-butenyl, 2-butenyl, and 3-butenyl.

In the present invention, "C2-4 alkynyl" includes ethynyl, 1-propynyl, 2-propynyl, 1-butynyl, 2-butynyl, and 3-butynyl.

In the present invention, "C1-4 alkoxy" includes methoxy, ethoxy, propoxy, isopropyloxy, butoxy, isobutoxy, sec-butoxy, and tert-butoxy.

In the present invention, "C3-6 cycloalkyl" in the "C3-6 cycloalkyl" and "-O-(C3-6 cycloalkyl)" includes cyclopropyl, cyclobutyl, cyclopentyl, and cyclohexyl.

In the present invention, "C5-6 monocyclic unsaturated carbocyclic ring" in the "C5-6 monocyclic unsaturated carbocyclic ring" and "-O-(C5-6 monocyclic unsaturated carbocyclic ring)" means C5-6 unsaturated or a partially saturated monocyclic carbocyclic ring, and for example, cyclopentene, cyclohexene, cyclopentadiene, cyclohexadiene, and benzene are given.

In the present invention, the "3-6 membered monocyclic heterocyclic ring" in the "3-6 membered monocyclic heterocyclic ring" and "-O-(3-6 membered monocyclic heterocyclic ring) means a 3-6 membered saturated, partially saturated or unsaturated monocyclic heterocyclic ring containing 1-2 heteroatom selected from a nitrogen atom, an oxygen atom and/or a sulfur atom which may be oxidized, and for example, oxirane, thiirane, aziridine, oxetane, thietane, azetidine, pyrroline, pyrrolidine, imidazoline, imidazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydrofuran, tetrahydrofuran, dihydropyran, tetrahydropyran, dihydrothiophene, tetrahydrothiophene, dihydrothiopyran, tetrahydrothiopyran, dihydroxazole, tetrahydroxazole(oxazolidine), dihydroisooxazole, tetrahydroisooxazole(isooxazolidine), dihydrothiazole, tetrahydrothiazole(thiazolidine), dihydroisothiazole, tetrahydroisothiazole(isothiazolidine), dihydroxazine, tetrahydroxazine, dihydrothiazine, tetrahydrothiazine, morpholine, thiomorpholine, oxathiane, pyrrole, imidazole, pyrazole, pyridine, pyrazine, pyrimidine, pyridazine, furan, pyran, thiophene, thiopyran, oxazole, isooxazole, thiazole, isothiazole, oxazine, and thiazine are given.

In the present invention, "an aromatic cyclic group which may be substituted" means a 5-12 membered monocyclic or bicyclic aromatic cyclic group being unsubstituted or having 1-3 substituent(s), which may contain 1-4 heteroatoms selected from a nitrogen atom, an oxygen atom and/or a sulfur atom which may be oxidized. As the aromatic cyclic group, an aromatic carbocyclic group or an aromatic heterocyclic group containing 1-4 heteroatoms selected from a nitrogen atom, an oxygen atom and/or a sulfur atom which may be oxidized, is included.

The "aromatic carbocyclic group" represents a 5-12 membered monocyclic or bicyclic aromatic carbocyclic group, and includes a monocyclic aromatic carbocyclic ring, a bicyclic aromatic carbocyclic ring, or a bicyclic fused ring formed of a monocyclic aromatic carbocyclic ring and an unsaturated or saturated monocyclic carbocyclic ring. For example, benzene, indene, indane, naphthalene, dihydronaphthalene, tetrahydronaphthalene, and azulene rings are given. However, in the case of an indene, indane, dihydronaphthalene, and tetrahydronaphthalene rings, a benzene ring among those rings binds to the following ring:

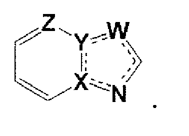

The "aromatic heterocyclic group containing 1-4 heteroatoms selected from a nitrogen atom, an oxygen atom and/or a sulfur atom which may be oxidized" represents a 5-12 membered monocyclic or bicyclic aromatic heterocyclic group containing 1-4 heteroatoms selected from a nitrogen atom, an oxygen atom and/or a sulfur atom which may be oxidized, and includes a monocyclic aromatic heterocyclic ring, a bicyclic aromatic heterocyclic ring, a bicyclic fused ring formed of a monocyclic aromatic heterocyclic ring and an unsaturated or saturated monocyclic carbocyclic ring, a bicyclic fused ring formed of a monocyclic aromatic carbocyclic ring and an unsaturated or saturated monocyclic heterocyclic ring, or a bicyclic fused ring formed of a monocyclic aromatic heterocyclic ring and an unsaturated or saturated monocyclic heterocyclic ring are included therein. For example, pyrrole, imidazole, triazole, pyrazole, pyridine, pyrazine, pyrimidine, pyridazine, furan, thiophene, oxazole, isooxazole, thiazole, isothiazole, furazan, oxadiazole, thiadiazole, indole, isoindole, benzofuran, isobenzofuran, benzothiophene, isobenzothiophene, indazole, quinoline, isoquinoline, purine, phthalazine, pteridine, naphthylidine, quinoxaline, quinazoline, cinnoline, benzooxazole, benzothiazole, benzoimidazole, benzofurazan, benzothiadiazole, benzotriazole, indoline, isoindoline, dihydrobenzofuran, dihydroisobenzofuran, dihydrobenzothiophene, dihydroisobenzothiophene, chromene, chromane, isochromane, tetrahydroquinoline, dihydroquinoline, tetrahydroisoquinoline, dihydroisoquinoline, tetrahydroquinoxaline, dihydroquinoxaline, tetrahydroquinazoline, dihydroquinazoline, and dioxaindan rings are given. However, in the case of the indole, isoindole, benzofuran, isobenzofuran, benzothiophene, isobenzothiophene, indazole, phthalazine, quinoxaline, quinazoline, cinnoline, benzooxazole, benzothiazole, benzoimidazole, benzofurazan, benzothiadiazole, benzotriazole, indoline, isoindoline, dihydrobenzofuran, dihydroisobenzofuran, dihydrobenzothiophene, dihydroisobenzothiophene, chromene, chromane, isochromane, and dioxaindan rings, a benzene ring among those rings, or in the case of tetrahydroquinoline, dihydroquinoline, tetrahydroisoquinoline, dihydroisoquinoline, tetrahydroquinoxaline, dihydroquinoxaline, tetrahydroquinazoline, and dihydroquinazoline rings, a pyridine, pyrimidine or pyrazine ring among those rings binds to the following ring:

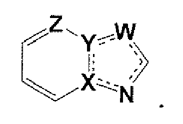

In the present invention, as the "substituents" in the aromatic cyclic group represented by the "aromatic cyclic group which may be substituted", (1) C1-15 alkyl which may be substituted, (2) C2-15 alkenyl which may be substituted, (3) C2-15 alkynyl which may be substituted, (4) hydroxy which may be protected, (5) mercapto which may be protected, (6) amino which may be protected, (7) carbamoyl which may be protected, (8) sulfamoyl which may be protected, (9) carboxyl which may be protected, (10) sulfo (-SO₃H) which may be protected, (11) sulfino (-SO₂H) which may be protected, (12) nitro, (13) cyano, (14) amidino, (15) imino, (16) a halogen atom, (17) a cyclic group which may be substituted, (18) C1-7 acyl, (19) oxo, and (20) thioxo are given. Those substituents may be arbitrary substituted at 1-3 substitutable positions.

In the present invention, C1-15 alkyl which may be substituted represents straight or branched C1-15 alkyl which may be substituted, and for example, methyl, ethyl, n-propyl, isopropyl, n-butyl, isobutyl, sec-butyl, tert-butyl, pentyl, hexyl, heptyl, octyl, nonyl, decyl, undecyl, dodecyl, tridecyl, tetradecyl, or pentadecyl which may be substituted is given.

In the present invention, C2-15 alkenyl which may be substituted represents straight or branched C2-15 alkenyl having 1-3 double bonds and which may be substituted, and for example, vinyl, propenyl, butenyl, pentenyl, hexenyl, hexadienyl, heptenyl, heptadienyl, octenyl, octadienyl, nonenyl, nonadienyl, decenyl, decadienyl, undecenyl, dodecenyl, tridecenyl, tetradecenyl, and pentadecenyl which may be substituted are given.

In the present invention, C2-15 alkynyl which may be substituted represents straight or branched C2-15 alkynyl having 1-3 triple bonds and which may be substituted, and for example, ethynyl, propynyl, butynyl, pentynyl, hexynyl, hexadynyl, heptynyl, heptadynyl, octynyl, octadynyl, nonynyl, decynyl, undecynyl, dodecynyl, tridecynyl, tetradecynyl, and pentadecynyl which may be substituted are given.

In the present invention, "C1-15 alkyl which may be substituted", "C2-15 alkenyl which may be substituted" and "C2-15 alkynyl which may be substituted" each represent "C1-15 alkyl which is substituted by a substituent(s) or unsubstituted", "C2-15 alkenyl which is substituted by a substituent(s) or unsubstituted" and "C2-15 alkynyl which is substituted by a substituent(s) or unsubstituted", and as the "substituent(s)", a group selected from the following substituent group A is given. Those substituents may be substituted at 1-4 substitutable positions.

The substituent group A represents (1) a halogen atom, (2) -CF₃, (3) -OCF₃, (4) cyano, (5) nitro, (6) C1-6 alkyl, C2-6 alkenyl, C2-6 alkynyl, or hydroxy which may be protected by a cyclic group or a protective group having desorption property, (7) C1-7 acyl, (8) C1-6 alkyl, C2-6 alkenyl, C2-6 alkynyl, or carboxyl which may be protected by a cyclic group, (9) C1-6 alkyl, C2-6 alkenyl, C2-6 alkynyl, or carbamoyl which may be protected by a cyclic group, (10) C1-6 alkyl, C2-6 alkenyl, C2-6 alkynyl, or mercapto which may be protected by a cyclic group, (11) NR⁹R¹⁰, in which R⁹ represents (a) a hydrogen atom, (b) C1-6 alkyl, (c) C2-6 alkenyl, (d) C2-6 alkynyl, or (e) a cyclic group; R¹⁰ represents (a) a hydrogen atom, (b) C1-6 alkyl, (c) C2-6 alkenyl, (d) C2-6 alkynyl, (e) -COR¹¹ in which R¹¹ represents a hydrogen atom, C1-6 alkyl, C2-6 alkenyl, C2-6 alkynyl, or a cyclic group; (f) -COOR¹¹ in which R¹¹ represent the same meaning described above; or (g) -CON(R⁹)₂ in which R⁹ each independently represent the same meaning described above, (12) -S(O)ₙR¹² in which n represents the same meaning described above; R¹² represents a hydrogen atom, C1-6 alkyl, C2-6 alkenyl, C2-6 alkynyl, or a cyclic group, (13) -COR¹¹ in which R¹¹ represents the same meaning described above, and (14) a cyclic group which may be substituted.

Further, C1-6 alkyl, C2-6 alkenyl, and C2-6 alkynyl in the substituent group A may be substituted by a group selected from a substituent group B, the cyclic group described in (14) of the substituent group A may be substituted by a group selected from a substituent group C. Those substituents may be substituted at 1-5 substitutable positions.

The substituent group B represents (1) C1-6 alkoxy, (2) C1-6 alkylthio, (3) a halogen atom, (4) hydroxy which may be protected by C1-6 alkyl, C2-6 alkenyl, C2-6 alkynyl, a cyclic group, a cyclic group-C1-6 alkyl or a protective group having desorption property, (5) CF₃, (6) OCF₃, (7) nitro, (8) cyano, (9) carboxyl, (10) (C1-6 alkoxy)carbonyl, (11) benzyloxycarbonyl, (12) mercapto, (13) amino, (14) C1-6 alkylamino, (15) di (C1-6 alkyl)amino, (16) carbamoyl, (17) N-(C1-6 alkyl) carbamoyl, (18) N,N-di(C1-6 alkyl) carbamoyl, (19) sulfamoyl, (20) N-(C1-6 alkyl)sulfamoyl, (21) N,N-di(C1-6 alkyl)sulfamoyl, (22) C1-7 acyl, and (23) a cyclic group which may be substituted by a group selected from a substituent group D.

As the substituent group C, (1) C1-6 alkyl, (2) C2-6 alkenyl, (3) C2-6 alkynyl, (4) hydroxy which may be protected by C1-6 alkyl, C2-6 alkenyl, C2-6 alkynyl, a cyclic group or a protective group having desorption property, (5) mercapto which may be protected by C1-6 alkyl, C2-6 alkenyl, C2-6 alkynyl or the cyclic group, (6) amino which may be protected by 1-2 groups selected from C1-6 alkyl, C2-6 alkenyl, C2-6 alkynyl and the cyclic group, (7) carbamoyl which may be protected by 1-2 groups selected from C1-6 alkyl, C2-6 alkenyl, C2-6 alkynyl, or the cyclic group, (8) sulfamoyl which may be protected by 1-2 groups selected from C1-6 alkyl, C2-6 alkenyl, C2-6 alkynyl, or the cyclic group, (9) carboxyl which may be protected by C1-6 alkyl, C2-6 alkenyl, C2-6 alkynyl or the cyclic group, (10) nitro, (11) cyano, (12) amidino, (13) a halogen atom, (14) CF₃, (15) OCF₃, (16) C1-7 acyl, (17) oxo, and (18) thioxo are given. Further, C1-6 alkyl, C2-6 alkenyl, and C2-6 alkynyl in the substituent group C may be substituted with a group selected from the substituent group B, and a cyclic group included in the substituent group C may be substituted by a group selected from a substituent group D.

The substituent group D represents (1) C1-6 alkyl, (2) C2-6 alkenyl, (3) C2-6 alkynyl, (4) C1-6 alkoxy, (5) C1-6 alkylthio, (6) a halogen atom, (7) CF₃, (8) OCF₃, (9) nitro, (10) cyano, (11) hydroxy which may be protected by C1-6 alkyl, C2-6 alkenyl, C2-6 alkynyl, the cyclic group, the cyclic group-C1-6 alkyl, or a protective group having desorption property, (12) carboxyl, (13) (C1-6 alkoxy)carbonyl, (14) benzyloxycarbonyl, (15) mercapto, (16) amino, (17) C1-6 alkylamino, (18) di (C1-6 alkyl) amino, (19) carbamoyl, (20) N-(C1-6 alkyl)carbamoyl, (21) N,N-di(C1-6 alkyl) carbamoyl, (22) sulfamoyl, (23) N-(C1-6 alkyl) sulfamoyl, (24) N,N-di(C1-6 alkyl)sulfamoyl, (25) C1-7 acyl, (26) oxo, and (27) thioxo.

In the present invention, as the hydroxy which may be protected, for example, (a) C1-15 alkyl which may be substituted, (b) C2-15 alkenyl which may be substituted, (c) C2-15 alkynyl which may be substituted, (d) a cyclic group which may be substituted, or (e) hydroxy protected by a protective group having desorption property or hydroxy which is not protected is given. In this case, as the protective group having desorption property, for example, trityl, methoxymethyl(MOM), 1-ethoxyethyl (EE), methoxyethoxymethyl (MEM), 2-tetrahydropyranyl (THP), trimethylsilyl (TMS), triethylsilyl (TES), t-butyldimethylsilyl (TBDMS), t-butyldiphenylsilyl (TBDPS), acetyl (Ac), pivaloyl, benzoyl, benzyl (Bn), p-methoxybenzyl, allyloxycarbonyl (Alloc), 2,2,2-trichloroethoxycarbonyl (Troc) are given. Further, hydroxy protected by C1-15 alkyl which may be substituted means C1-15 alkoxy which may be substituted.

In the present invention, mercapto which may be protected represents (a) C1-15 alkyl which may be substituted, (b) C2-15 alkenyl which may be substituted, (c) C2-15 alkynyl which may be substituted, or (d) mercapto protected by a cyclic group which may be substituted or unprotected mercapto.

In the present invention, amino which may be protected represents amino protected by the following 1-2 protective groups or unprotected amino. As the protective groups for the amino, (a) C1-15 alkyl which may be substituted, (b) C2-15 alkenyl which may be substituted, (c) C2-15 alkynyl which may be substituted, (d) a cyclic group which may be substituted, (e) -COR¹³ in which R¹³ represents (aa) a hydrogen atom, (bb) C1-15 alkyl, C2-15 alkenyl or C2-15 alkynyl which may be substituted, or (cc) a cyclic group which may be substituted, (f) -COOR¹³ in which R¹³ represents the same meaning described above, and (g) -CON(R¹⁴)₂ in which R¹⁴ each independently represent, (aa) a hydrogen atom or (bb) C1-15 alkyl, C2-15 alkenyl or C2-15 alkynyl which may be substituted are given.

In the present invention, as the "protective group" in the "carbamoyl which may be protected", "sulfamoyl which may be protected", "carboxyl which may be protected", "sulfo which may be protected", and "sulfino which may be protected", (a) C1-15 alkyl which may be substituted, (b) C2-15 alkenyl which may be substituted, (c) C2-15 alkynyl which may be substituted, or (d) a cyclic group which may be substituted are given.

In the present invention, C1-7 acyl represents, for example, formyl, acetyl, propanoyl, pivaloyl, or benzoyl.

In the present invention, the cyclic group represents a carbocyclic group or a heterocyclic group.

The carbocyclic group represents a C3-12 totally saturated, a partially saturated, or a totally unsaturated monocyclic or bicyclic carbocyclic group, and, for example, cyclopropane, cyclobutane, cyclopentane, cyclohexane, cycloheptane, cyclopentene, cyclohexene, cycloheptene, cyalopentadiene, cyclohexadiene, cycloheptadiene, benzene, pentalene, perhydropentalene, azulene, perhydroazulene, indene, perhydroindene, indane, naphthalene, dihydronaphthalene, tetrahydronaphthalene, perhydronaphthalene, heptalene, and perhydroheptalene rings are given.

The heterocyclic group represents a 3-12 membered totally saturated, partially saturated, or totally unsaturated monocyclic or bicyclic heterocyclic group containing 1-4 heteroatoms selected from a nitrogen atom, an oxygen atom and/or a sulfur atom which may be oxidized, and, for example, oxirane, thiirane, aziridine, oxetane, thietane, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydrofuran, tetrahydrofuran, dihydropyran, tetrahydropyran, dihydrooxepine, tetrahydrooxepine, perhydrooxepine, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzooxazole, dihydrobenzothiazole, perhydrobenzothiazole, dihydrobenzimidazole, perhydrobenzimidazole, dihydrobenzazepine, tetrahydrobenzazepine, dihydrobenzodiazepine, tetrahydrobenzodiazepine, benzodioxepane, dihydrobenzoxazepine, tetrahydrobenzoxazepine, pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, benzoxazole, benzothiazole, benzimidazole, chromene, benzoxepine, benzoxazepine, benzoxadiazepine, benzothiepine, benzothiazepine, benzothiadiazepine, benzoazepine, benzodiazepine, benzofurazan, benzothiadiazole and benzotriazole rings are given.

In the present invention, the cyclic group which may be substituted represents a carbocyclic group or a heterocyclic group which each may be substituted with 1-5 groups selected from a substituent group C. As the carbocyclic group and a heterocyclic group, the carbocyclic groups and heterocyclic groups are given.

The C1-15 alkyl which may be substituted, C2-15 alkenyl which may be substituted, C2-15 alkynyl which may be substituted, and the cyclic group which may be substituted in the "protective group" described above each represent the same meanings described above.

In the present invention, C1-6 alkyl represents, for example, methyl, ethyl, n-propyl, isopropyl, n-butyl, isobutyl, sec-butyl, tert-butyl, pentyl, hexyl, and isomers thereof.

In the present invention, C2-6 alkenyl represents, for example, vinyl, propenyl, butenyl, pentenyl, hexenyl, hexadienyl, and isomers thereof.

In the present invention, C2-6 alkynyl represents, for example, ethynyl, propynyl, butynyl, pentynyl, hexynyl, hexadiynyl and isomers thereof.

In the present invention, hydroxy protected with C1-6 alkyl means C1-6 alkoxy.

In the present invention, C1-6 alkoxy represents, for example, methoxy, ethoxy, propoxy, isopropoxy, butoxy, isobutoxy, sec-butoxy, tert-butoxy, pentyloxy, or hexyloxy.

In the present invention, C1-15 alkoxy represents straight or branched C1-15 alkoxy, and, for example, methoxy, ethoxy, propoxy, isopropoxy, butoxy, isobutoxy, sec-butoxy, tert-butoxy, pentyloxy, hexyloxy, heptyloxy, octyloxy, nonyloxy, decyloxy, undecyloxy, dodecyloxy, tridecyloxy, tetradecyloxy, or pentadecyloxy is given.

In the present invention, C1-6 alkylthio represents, for example, methylthio, ethylthio, propylthio, isopropylthio, n-butylthio, isobutylthio, sec-butylthio, tert-butylthio, pentylthio, or hexylthio.

In the present invention, (C1-6 alkoxy)carbonyl represents, for example, methoxycarbonyl, ethoxycarbonyl, propylcarbonyl, isopropylcarbonyl, butoxycarbonyl, isobutoxycarbonyl, sec-butoxycarbonyl, tert-butoxycarbonyl, pentyloxycarbonyl, or hexyloxycarbonyl.

In the present invention, C1-6 alkylamino represents, for example, methylamino, ethylamino, propylamino, isopropylamino, n-butylamino, isobutylamino, sec-butylamino, tert-butylamino, pentylamino, or hexylamino.

In the present invention, di(C1-6 alkyl)amino represents, for example, dimethylamino, diethylamino, dipropylamino, diisopropylamino, dibutylamino, dipentylamino, dihexylamino, or N-methyl-N-ethylamino.

In the present invention, N-(C1-6 alkyl)carbamoyl represents, for example, N-methylcarbamoyl, N-ethylcarbamoyl, N-propylcarbamoyl, N-isopropylcarbamoyl, N-butylcarbamoyl, N-isobutylcarbamoyl, N-(sec-butyl)carbamoyl, N-(tert-butyl)carbamoyl, N-pentylcarbamoyl, or N-hexylcarbamoyl.

In the present invention, N,N-di(C1-6 alkyl)carbamoyl represents, for example, N,N-dimethylcarbamoyl, N,N-diethylcarbamoyl, N,N-dipropylcarbamoyl, N,N-diisopropylcarbamoyl, N,N-dibutylcarbamoyl, N,N-dipentylcarbamoyl, N,N-dihexylcarbamoyl, or N-methyl-N-ethylcarbamoyl.

In the present invention, N-(C1-6 alkyl)sulfamoyl represents, for example, N-methylsulfamoyl, N-ethylsulfamoyl, N-propylsulfamoyl, N-isopropylsulfamoyl, N-butylsulfamoyl, N-isobutylsulfamoyl, N-(sec-butyl)sulfamoyl, N-(tert-butyl)sulfamoyl, N-pentylsulfamoyl, or N-hexylsulfamoyl.

In the present invention, N,N-di(C1-6 alkyl)sulfamoyl represents, for example, N,N-dimethylsulfamoyl, N,N-diethylsulfamoyl, N,N-dipropylsulfamoyl, N,N-diisopropylsulfamoyl, N,N-dibutylsulfamoyl, N,N-dipentylsulfamoyl, N,N-dihexylsulfamoyl, or N-methyl-N-ethylsulfamoyl.

In the present invention, the cyclic group-C1-6 alkyl represents a carbocyclic group-C1-6 alkyl or a heterocyclic group-C1-6 alkyl, each representing C1-6 alkyl substituted with one carbocyclic group or C1-6 alkyl substituted with one heterocyclic group. The carbocyclic group, heterocyclic group and C1-6 alkyl each represent the same meanings described above.

In the present invention, as the preferred compound represented by the formula (I), a compound represented by the formula (1-A), formula (I-B), formula (I-C) or formula (I-D) is given: 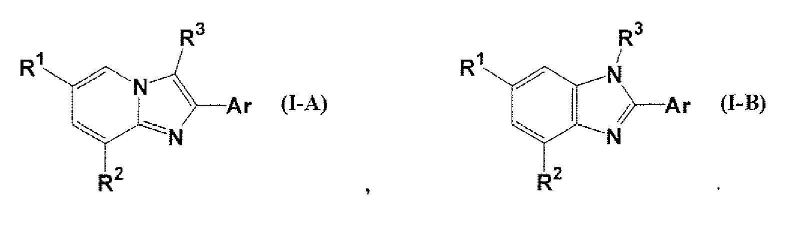 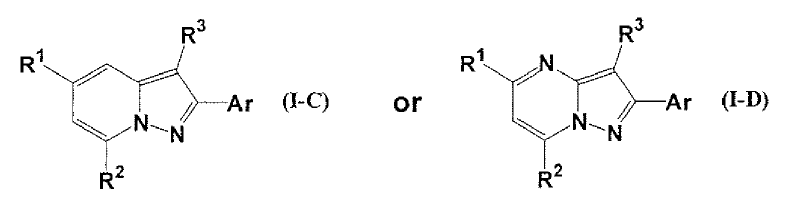 wherein all symbols represent the same meanings described above. As the specific compound, compounds as described in Examples below are given.

In the present invention, it is preferred that R¹ has a branched chain. R¹ preferably includes (1) C3-10 branched alkyl which may be substituted, or (2) -NR⁴R⁵ in which all symbols represent the same meanings described above. In the case of C3-10 branched alkyl which may be substituted, C3-10 branched alkyl whose branch source is a carbon atom at position 1, is more preferred. Particularly preferred is C3-8 branched alkyl which may be substituted, and C3-8 branched alkyl whose branch source is a carbon atom at position 1, is further preferred. In addition, C3-10 unsubstituted branched alkyl chain is also preferred. Specifically, isopropyl, sec-butyl, tert-butyl, 1-methylbutyl, 1-ethylpropyl, 1,1-dimethylpropyl, 1,2-dimethylpropyl, 1-methylpentyl, 1-ethylbutyl, 1-ethyl-1-methylpropyl, 1-ethyl-2-methylpropyl, 1,1-dimethylbutyl, 1,2-dimethylbutyl, 1,3-dimethylbutyl, 1-methylhexyl, 1-ethylpentyl, 1-propylbutyl, 1,2-dimethylpentyl, 1,3-dimethylpentyl, 1,4-dimethylpentyl, 1-ethyl-1-methylbutyl, 1-methyl-2-ethylbutyl, 1-ethyl-2-methylbutyl, 1-ethyl-3-methylbutyl, 1,1-dimethylpentyl, 1,1,3-trimethylbutyl, 1,1-diethylpropyl, 1-methylheptyl, 1-ethylhexyl, 1-propylpentyl, 1,5-dimethylhexyl, 1-ethyl-4-methylpentyl, 1-propyl-3-methylbutyl, 1,1-dimethylhexyl, 1-ethyl-1-methylpentyl, and 1,1-diethylbutyl are given. Particularly preferably, 1-methylbutyl, 1-ethylpropyl, 1-methylpentyl, 1-ethylbutyl, 1-methylhexyl, 1-ethylpentyl, 1-propylbutyl, 1-methylheptyl, 1-ethylhexyl, and 1-propylpentyl are given. Besides, it is also preferred that in the C3-10 branched alkyl, the number of carbon atoms of two alkyl groups branched from the carbon at position 1 is the same.

In the present invention, in (1) C3-10 branched alkyl which may be substituted, or (2) -NR⁴R⁵ in which all symbols represent the same meanings described above, being a preferred group as R¹, (1) C3-10 unsubstituted branched alkyl, or C3-10 branched alkyl substituted with 1-2 substituent(s), and (2) - NR⁴R⁵ in which R⁴ and R⁵ each independently represent, C1-6 unsubstituted alkyl, C1-6 alkyl substituted with 1-2 substituent(s), or R⁴ represents a hydrogen atom, and R⁵ represents unsubstituted C3-6 branched alkyl, or C3-6 branched alkyl substituted with 1-2 substituent(s) are more preferred. As the preferred substituents in the "substituted C3-10 branched alkyl", "substituted C1-6 alkyl" and "substituted C3-6 branched alkyl", C1-4 alkoxy, -CF₃, -OCF₃, cyclopropyl, cyclobutyl, and hydroxyl are given, and those substituents may be substituted at 1-2 substitutable positions.

In the present invention, preferably R² and R³ each independently represent a hydrogen atom, C1-4 alkyl which may be substituted with hydroxy which may be protected, nitrile, COOR⁶, CONR⁷R⁸, COR¹⁰¹, or S (O)ₙR¹⁰², in which all symbols represent the same meanings described above. As R², R²⁻¹ namely, a hydrogen atom, unsubstituted C1-4 alkyl, or nitrile is more preferred. More preferred as R³ is R³⁻¹, namely, a hydrogen atom, C1-4 alkyl which may be substituted with hydroxy which may be protected, nitrile, COOR⁶ where as R⁶, C1-4 alkyl is preferred, CONR⁷R⁸ in which all symbols represent the same meanings described above, COR¹⁰¹ in which all symbols represent the same meanings described above, or S(O)ₙR¹⁰² in which all symbols represent the same meanings described above. Particularly preferred as R³ is R³⁻¹⁻¹, namely, C1-4 unsubstituted alkyl, or CONR⁷R⁸ in which all symbols represent the same meanings described above.

In the present invention, Ar preferably includes, each having 1-3 substituent(s), a 5-12 membered monocyclic aromatic carbocyclic ring, a bicyclic aromatic carbocyclic ring, or a bicyclic fused ring formed of a monocyclic aromatic carbocyclic ring and an unsaturated or saturated monocyclic carbocyclic ring, or, each containing 1-4 heteroatoms selected from a nitrogen atom, an oxygen atom and/or a sulfur atom which may be oxidized, a 5-12 membered monocyclic aromatic heterocyclic ring, a bicyclic aromatic heterocyclic ring, or a bicyclic fused ring formed of a monocyclic aromatic heterocyclic ring and an unsaturated or saturated monocyclic carbocyclic ring, a bicyclic fused ring formed of a monocyclic aromatic carbocyclic ring and an unsaturated or a saturated monocyclic heterocyclic ring, or a bicyclic fused ring formed of monocyclic aromatic heterocyclic ring and an unsaturated or saturated monocyclic heterocyclic ring, and more preferably, Ar includes, each having 1-3 substituent(s), benzene, indene, indane, naphthalene, dihydronaphthalene, tetrahydronaphthalene, azulene, pyrrole, imidazole, triazole, pyrazole, pyridine, pyrazine, pyrimidine, pyridazine, furan, thiophene, oxazole, isooxazole, thiazole, isothiazole, furazan, oxadiazole, thiadiazole, indole, isoindole, benzofuran, isobenzofuran, benzothiophene, isobenzothiophene, indazole, quinoline, isoquinoline, purine, phthalazine, pteridine, naphthylidine, quinoxaline, quinazoline, cinnoline, naphthylidine, benzoxazole, benzothiazole, benzimidazole, benzofurazan, benzothiadiazole, benzotriazole, indoline, isoindoline, dihydrobenzofuran, dihydroisobenzofuran, dihydrobenzothiophene, dihydroisobenzothiophene, chromene, chromane, isochromane, tetrahydroquinoline, dihydroquinoline, tetrahydroisoquinoline, dihydroisoquinoline, tetrahydroquinoxaline, dihydroquinoxaline, tetrahydroquinazoline, dihydroquinazoline, or dioxaindane ring, and further preferably, Ar includes, each having 1-3 substituent(s), benzene, indane, pyridine, pyrimidine, dioxaindan ring. Particularly preferred is a benzene ring having 1-3 substituent(s).

In the present invention, as the more preferred compound represented by the formula (I), a compound represented by the formula (I-A-1), formula (I-B-1), or formula (I-C-1) is given: 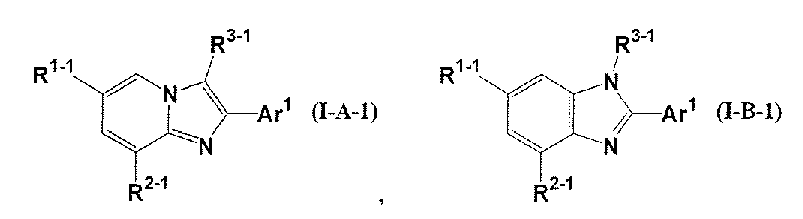 or 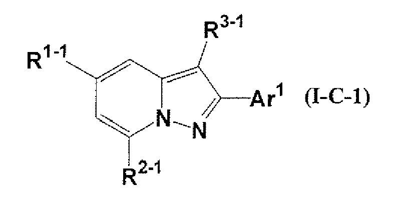 wherein all symbols represent the same meanings described above.

In the present invention, as the more preferred compound represented by the formula (I-A-1), the following compound (I-A-1-1) is given: 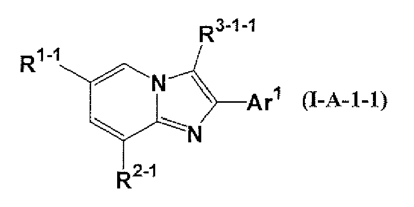 wherein all symbols represent the same meanings described above.

As the preferred substituents for Ar or Ar¹ described above, (1) C1-15 alkyl which may be substituted, (2) C1-15 alkenyl which may be substituted, (3) hydroxy which may be protected, (4) mercapto which may be protected, (5) amino which may be protected, (6) carbamoyl which may be protected, (7) carboxyl which may be protected, (8) sulfo which may be protected, (9) sulfino which may be protected, (10) cyano, (11) a halogen atom, and (12) a cyclic group which may be substituted are given. Particularly preferred are (1) C1-6 alkyl, (2) C2-6 alkenyl, (3) unsubstituted hydroxy, or hydroxy protected by C1-6 alkyl which may be substituted, or a protective group having desorption property, in which C1-6 alkoxy and trifluoromethoxy are particularly preferred, (4) unsubstituted mercapto, or mercapto protected by C1-6 alkyl which may be substituted, or a protective group having desorption property, in which particularly C1-6 alkylthio is preferred, (5) carboxyl, or carboxyl protected by C1-6 alkyl, or benzyl, (6) cyano, (7) a halogen atom, (8) a cyclic group which may be substituted, and (9) CF₃. Further, it is preferred that those substituents may be substituted at 1, 2 or 3 substitutable positions of the ring represented by Ar. In particular, in the case where Ar is a 6 membered monocyclic ring, specifically, a benzene or pyridine ring, in the following ring: 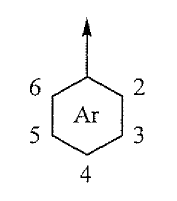 wherein the above ring; 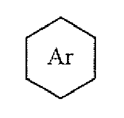 represents a benzene ring or a pyridine ring, and an arrow; 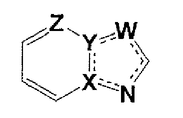 binds to the above ring, the substitution is preferably carried out at (1) position 2, (2) position 3, (3) position 4, (4) positions 2 and 4, (5) positions 2, 4, and 5, or (5) positions 2, 4, and 6.

Unless otherwise specified, all isomers are included in the present invention. For example, alkyl, alkenyl, alkynyl, alkoxy, alkylthio, alkylene, alkenylene and alkynylene include straight and branched isomers. In addition, isomers based on double bond, ring, fused ring (E, Z, cis, trans), isomers resulting from the presence of asymmetric carbon(s) (R, S-configuration, α, β-configuration, enantiomers, diastereomers), optically active compounds having optical rotation (D, L, d, 1-configuration), polar compounds obtained by chromatographic separations (more polar compound, less polar compound), equilibrium compounds, rotational isomers, the mixtures thereof at any ratio, racemic mixtures are included in the present invention.

In the present invention, as is apparent to the one skilled in the art, unless otherwise indicated, 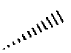
the mark shows that the bond is on the other side of paper (α-configuration), 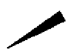
the mark shows that the bond is in front of paper (β-configuration), 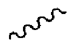
the mark shows that the bond is α-configuration or β-configuration, and 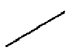
the mark shows that the bond is a mixture of α-configuration and β-configuration.

### [Salts]

The compound represented by the formula (I) may be converted into a salt by known methods. As the salt, pharmaceutically acceptable salts are preferred.

As the salt, alkali metal salt, alkaline earth metal salt, ammonium salt, amine salt, acid addition salt, etc. are given.

Aqueous salt is preferred as the salt. As appropriate salts thereof, salts of alkali metals such as potassium and sodium; salts of alkaline-earth metals such as calcium and magnesium; ammonium salts such as tetramethylammonium; and salts of pharmaceutically acceptable organic amines such as triethylamine, methylamine, dimethylamine, cyclopentylamine, benzylamine, phenethylamine, piperidine, monoethanolamine, diethanolamine, tris(hydroxymethyl)aminomethane, lysine, arginine, and N-methyl-D-glucamine are given.

Aqueous salts are preferred as the acid addition salts. As appropriate salts thereof, for example, salts of inorganic acids such as hydrochloride, hydrobromide, hydroiodide, sulfate, phosphate, nitrate, or salts of organic acid such as acetate, lactate, tartrate, benzoate, citrate, methanesulfonate, ethanesulfonate, trifluoroacetate, benzenesulfonate, toluenesulfonate, isethionate, glucuronate, or gluconate are given.

Further, N-oxide is included in the salts. The compound of the present invention may be converted into an N-oxide compound by any methods. The N-oxide is the compound in which a nitrogen atom of the compound represented by the formula (I) is oxidized.

The compound represented by the formula (I) and its salt may be replaced with a solvate.

The solvate is preferably nontoxic and aqueous. As appropriate solvates, for example, water or alcohol-based solvents such as ethanol are given.

### [Prodrug]

The prodrug of a compound represented by the formula (I) refers to a compound which is converted into the compound represented by the formula (I) through reaction with an enzyme, a gastric acid, or the like, in the living body. The prodrug of a compound represented by the formula (I) may be exemplified by the compounds represented by the formula (I) where an amino group is contained, in which the amino group has been allowed to undergo acylation, alkylation or phosphorylation (for example, the compounds represented by the formula (I) in which the amino group has been allowed to undergo eicosanoylation, alanylation, pentylaminocarbonylation, (5-methyl-2-oxo-1,3-dioxolen-4-yl)methoxycarbonylation, tetrahydrofuranylation, pyrrolizylmethylation, pivaloyloxymethylation, acetoxymethylation, tert-butylation, etc.); the compounds represented by the formula (I) where a hydroxy group is contained, in which the hydroxy group has been allowed to undergo acylation, alkylation, phosphorylation or boration (for example, the compounds represented by the formula (I) in which the hydroxy group has been allowed to undergo acetylation, palmitoylation, propanoylation, pivaloylation, succinylation, fumarylation, alanylation, dimethylaminomethylcarbonylation, etc.); the compounds represented by the formula (I) where a carboxyl group is contained, in which the carboxyl group has been allowed to undergo esterification or amidation (for example, the compounds represented by the formula (I) in which the carboxyl group has been allowed to undergo ethyl-esterification, isopropyl-esterification, phenyl-esterification, carboxymethyl-esterification, dimethylaminomethyl-esterification, pivaloyloxymethyl-esterification, ethoxycarbonyloxyethyl-esterification, phthalidyl-esterification, (5-methyl-2-oxo-1,3-dioxolen-4-yl)methyl-esterification, cyclohexyloxycarbonylethyl-esterification, methyl-amidation, etc.); the compounds represented by the formula (I) where a carboxyl group is contained, in which the carboxyl group has been allowed to undergo replacement with a hydroxymethyl group, and the like. Those compounds can be produced in accordance with the per se known processes. Besides, the prodrug of the compound represented by the formula (I) may be any of the forms of hydrated or non-hydrated products.

### [Production Process for Compound of the Present Invention]

The compound of the present invention can be produced by the following method, for example.
(1) In the compound represented by the formula (I), the compound represented by the formula (I-A): 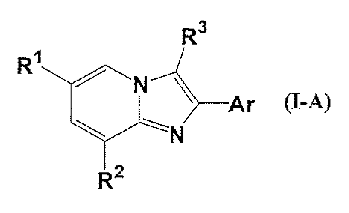 wherein all symbols represent the same meanings described above, can be produced through the reaction of the compound represented by the formula (II): 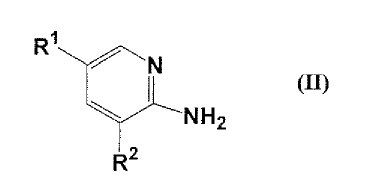 wherein all symbols represent the same meanings described above, with the compound represented by the formula (III): 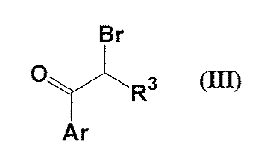 wherein all symbols represent the same meanings described above.

The above reaction is known, and the reaction is carried out, for example, by heating at 50-250°C in an organic solvent (for example, N-methylpyrrolidone, dimethylformamide, or isopropanol), or without the solvent. The heating may be carried out using a water bath, oil bath, sand bath, or microwave.

In the compound represented by the formula (I-A), in the case where R² or R³ is a compound which represents COOR⁶, CONR⁷R^{B} or nitrile, namely a compound represented by the formula (I-A-a): 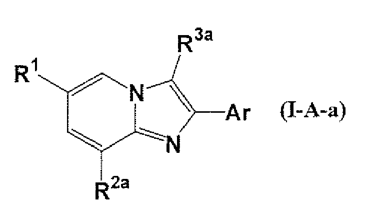 wherein R^{2a} and R^{3a} each independently represent R² and R³, but at least one thereof represents COOR⁶, CONR⁷R⁸ or nitrile, and the other symbols represent the same meanings described above, the formula (II-a): 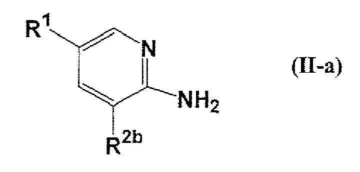 wherein R^{2b} represents C1-4 alkyl, C1-4 alkyl substituted with a halogen atom, C1-4 alkynyl, or COOR^{6a} in which R^{6a} represent C1-4 alkyl, and the other symbols represent the same meanings described above.) is reacted with a compound represented by the formula (III-a): 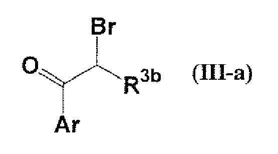 wherein R^{3b} represents C1-4 alkyl, C1-4 alkyl substituted with a halogen atom, C1-4 alkenyl, C1-4 alkynyl, or COOR^{6a} in which R^{6a} represents C1-4 alkyl, and the other symbols represent the same meanings described above, but, in the case where R^{2b} represents a group other than COOR^{6a}, R^{3b} represents COOR^{6a}, to produce the compound. Further, the compound may be produced by the followings: subjecting to a hydrolysis reaction; subsequently an obtained carboxylic acid is subjected to an amidation reaction together with (1) R⁷R⁸NH in which all symbols represent the same meanings described above; or is subjected to the amidation reaction with (2) NH₃ and successively dehydration reaction.

The reaction of the compound represented by the formula (II-a) with the compound represented by the formula (III-a) is carried out by the same process.

The hydrolysis reaction is known, and is carried out, for example, at 0-80°C by using hydroxide of an alkaline metal (sodium hydroxide, potassium hydroxide, lithium hydroxide, etc.), hydroxide of an alkaline earth metal (barium hydroxide, calcium hydroxide, etc.) or carbonate (sodium carbonate, potassium carbonate), or an aqueous solution thereof, or a mixture thereof in an organic solvent (methanol, ethanol, terahydrofuran, dioxan, etc.).

The amidation described above is known, and is carried out, for example, in an organic solvent (chloroform, methylene chloride, diethylether, tetrahydrofuran, etc.) or without solvent, by reacting with an acid halide agent (oxaryl chloride, thionyl chloride, etc.) at -20°C to reflux temperature, and the obtained acid halide is reacted, in the presence or absence of a base (pyridine, triethylamine, dimethylaniline, dimethylaminopyridine, diisopropylethylamine, etc) with R⁷R⁸NH in which all symbols represent the same meanings described above, in an organic solvent (chloroform, dichloromethan, diethylether, tetrahydrofuran, etc.) at 0-40°C. Further, the amidation reaction can be carried out by reacting the obtained acid halide, in an organic solvent (dioxan, tetrahydrofuran, etc.), by using alkaline solution (sodium hydrogencarbonate, aqueous sodium hydroxide, or the like) with R⁷R⁸NH in which all symbols represent the same meanings described above, at 0-40°C.

The anhydration reaction described above is known, and is carried out, for example, in an organic solvent (toluene, dimethylformamide, etc.) by using a dehydrating agent (phosphorus oxychloride, etc.) at room temperature to reflux temperature of solvent.

In the compound represented by the formula (I-A), in the case where R³ represents a compound which represents COR¹⁰¹, S(O)ₙR¹⁰² or C1-4 alkyl substituted with hydroxy which may be protected, namely, a compound represented by the formula (I-A-b) : 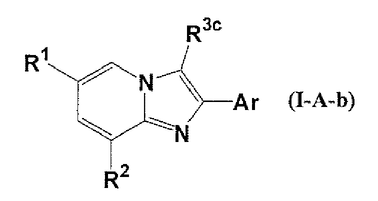 wherein, R^{3c} represents COR¹⁰¹, S(O)ₙR¹⁰², or C1-4 alkyl substituted with hydroxy which may be protected, and the other symbols represent the same meanings described above, a compound represented by the formula (II-b): 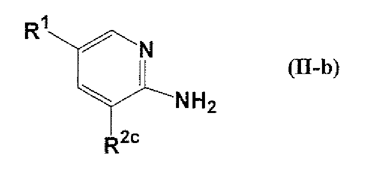 wherein R^{2c} represents C1-4 alkyl, C1-4 alkyl substituted with a halogen atom, C1-4 alkenyl, or C1-4 alkynyl, and the other symbols represent the same meanings described above, is reacted with a compound represented by the formula (III-b) 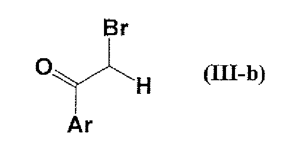 wherein all symbols represent the same meanings described above, and subsequently subjected to a halogenation reaction, and thereafter subsequently carried out (1) an acylation reaction; (2) a coupling reaction; or subjecting the compound obtained by the acylation reaction described above to a reduction reaction, thereby being capable of producing the compound.

The reaction of the compound represented by the formula (II-b) and the compound represented by the formula (III-b) is carried out by the same process with the reaction described above.

The halogenation reaction is known, and is carried out, for example, in an organic solvent (tetrahydrofuran, dioxan, dichloromethane, etc.) by reacting with a halogenation reagent (N-bromosuccinimide, bromine, chlorine, iodine, etc.) at 0-80°C.

The acylation reaction is known, and is carried out, for example, in an organic solvent (tetrahydrofuran, dimethoxyethan, etc.) or without solvent, by using a metalation reagent (isopropylmagnesium chloride, methylmagnesium chloride, t-butyllithium, sec-butyllithium, lithiumdiisopropylamido, etc.) and an acylation agent (acetyl chloride, dimethylformamide, etc.) at -20°C to reflux temperature.

The coupling reaction is known, and is carried out, for example, in an organic solvent (dimethylsulfoxide, dimethylformamide, dioxan, toluene, tetrahydrofuran, etc.) or without solvent, in the presence of copper catalyst (copper(I) iodide, copper(I) bromide, copper(I) chloride, copper acetate, etc.), amino acid (L-proline, D-proline, N-methylglycine, etc.), and alkaline hydride (sodium hydride, potassium hydride, lithium hydride, etc.), by reacting with sulfinic acid salt (methanesulfinic acid sodium, benzenesulfinic acid sodium, etc.) at room temperature to reflux temperature of solvent.

The reduction reaction is known, and is carried out, for example, in an organic solvent (methanol, ethanol, tetrahydrofuran, etc.), by using a reducing agent (sodium borohydride, diisopropyl aluminum hydride, lithium aluminium hydride, etc.) at -78°C to reflux temperature of solvent.
(2) In the compound represented by the formula (I), a compound represented by the formula (I-B): 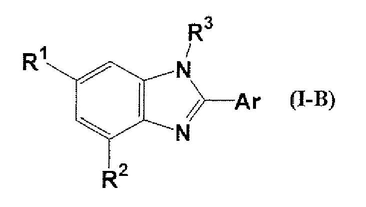 wherein all symbols represent the same meanings described above, can be produced by reacting a compound represented by the formula (IV): 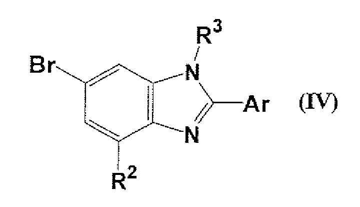 wherein all symbols represent the same meanings described above, with a compound represented by the formula (V):

   **R¹-ZnBr** **(V)**

   wherein all symbols represent the same meanings described above.
   The reaction described above is known, and is carried out, for example, in an organic solvent (tetrahydrofuran, dimethylether, dioxane, toluene, etc.), by using a copper catalyst (copper iodide, etc.) and a palladium catalyst ([1,1'-bis(diphenylphosphino)ferrocene]dichloropalladium, etc.) at room temperature to reflux temperature.
(3) In the compound represented by the formula (I), a compound represented by the formula (I-C): 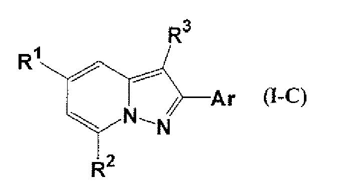
wherein all symbols represent the same meanings described above, can be produced by reacting a compound represented by the formula (VI): 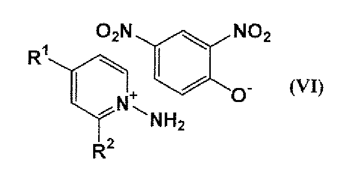 wherein all symbols represent the same meanings described above, with a compound represented by the formula (VII): 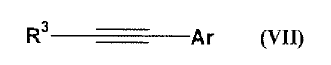 wherein all symbols represent the same meanings described above.

The reaction is known, and is carried out, for example, in an organic solvent (for example, dimethylsulfoxide, dimethylformamide, and methylene chloride), by using potassium carbonate or potassium hydrate at 0-40°C.

Further, a compound represented by the formula (I-C) can be produced: through the hydrolysis reaction using a compound represented by the formula (I-C-a): 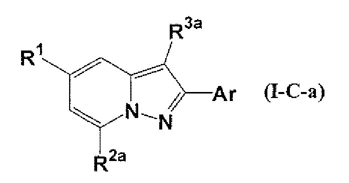 wherein R^{2a} and R^{3a} each independently represent the same meanings with R² and R³, but at least one thereof represents COOR^{6a} in which R^{6a} represents the same meaning described above, and the other symbols represent the same meanings described above; subsequently subjecting the obtained carboxylic acid to (1) amidation reaction with R⁷R⁸NH in which all symbols represent the same meanings described above, or subjecting to (2) amidation reaction with NH₃, and then subsequently carried out dehydration reaction. Further, the compound can be produced by, after the hydrolysis reaction described above, carrying out decarboxylation reaction, and subsequently halogenation reaction, and further alkylation or alkynylation reaction sequentially.

The hydrolysis reaction, amidation reaction, or dehydration reaction is carried out by the same process described above.

The decarboxylation reaction is known, and is carried out, for example, by using acid (hydrobromic acid, etc.) at 60°C to reflux temperature.

The halogenation reaction is known, and is carried out, for example, in an organic solvent (acetonitrile, etc.), by using N-bromosuccinimide at 0-40°C.

The alkylation reaction and alkynylation reaction are known, and carried out, for example, in an organic solvent (toluene, etc.), by using water, boronic acid (methylboronic acid, etc.), palladium catalyst (palladium acetate, etc.), and ligand (tricyclohexylphosphoine, etc.), a base (tripotassium phosphate, potassium carbonate, triethylamine, etc.) at room temperature to reflux temperature of solvent.

In the compound represented by the formula (I), a compound represented by the formula (I-D) can be produced by the combination of the known methods.

The starting material of the present invention can be produced in accordance with the known method. Compounds represented by the formula (II), (III), (IV), and (VI) can be produced, for example, by a process represented by the following reaction schemes.

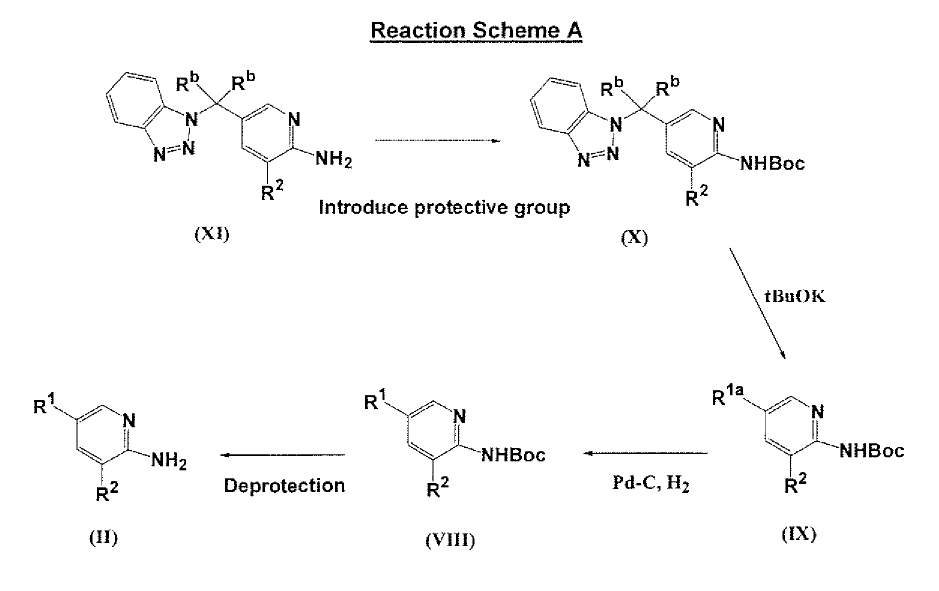

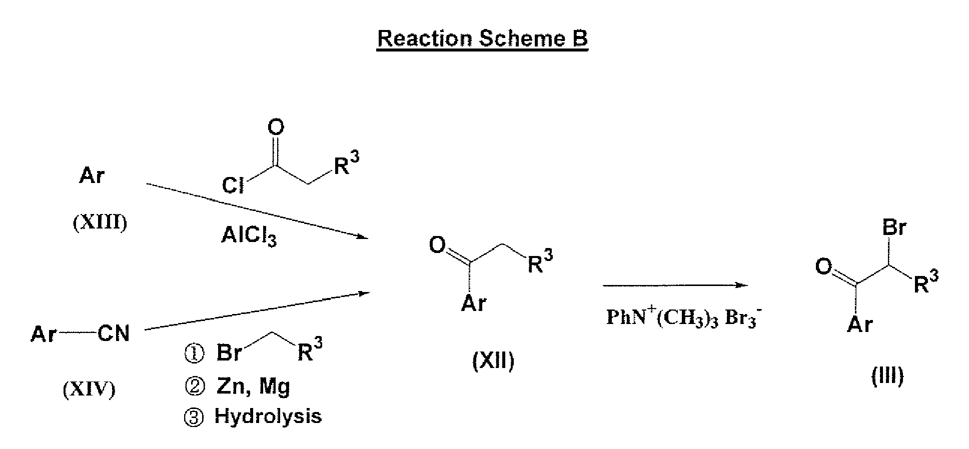

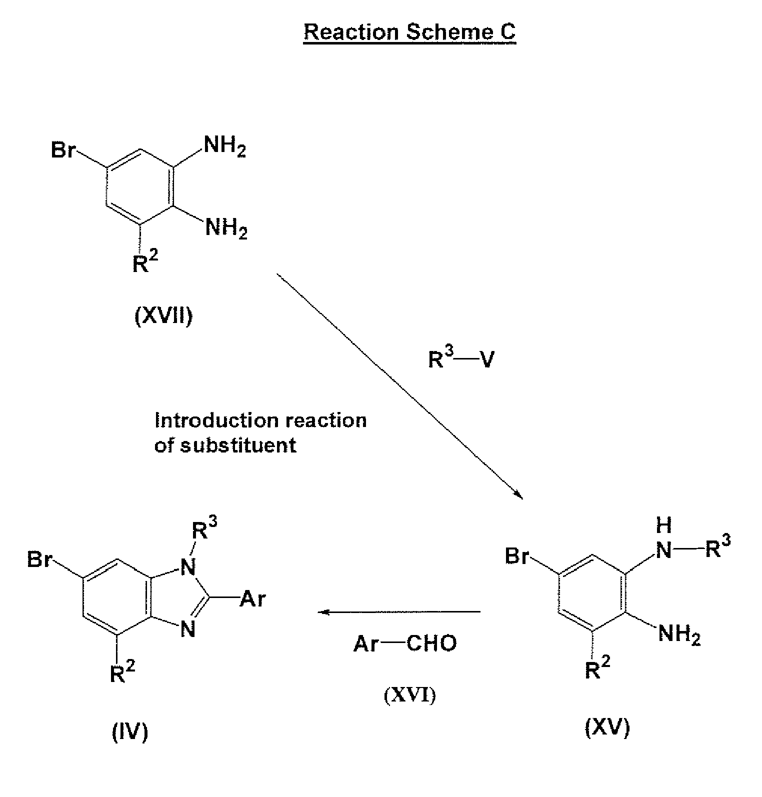

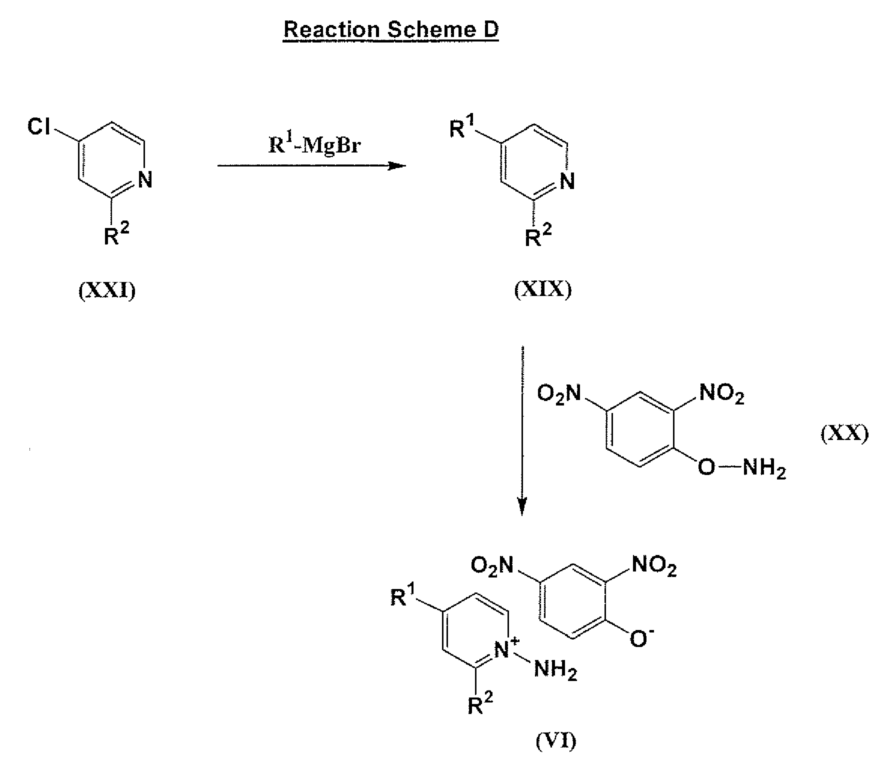

In the reaction schemes A, B, C, and D, R^{1a} is branched at a carbon atom on position 1, and is C3-10 branched alkenyl which may be substituted having one double bond at a carbon on position 1, R^{b} each independently represent C1-8 alkyl which may be substituted, but the total number of the carbon atoms of alkyl represented by two R^{b} is 9 in maximum, Boc represents t-butoxy carbonyl, R^{c} represents C1-4 alkyl, V represents a leaving group (for example, a halogen atom, or imidazolyl), and the other symbols represent the same meanings described above.

Further, the other starting material and the respective reagents in the present invention are per se known or can be produced in accordance with the known method.

In each reaction in the present specification, reaction products may be purified by general purification techniques, for example, by distillation under atmospheric or reduced pressure, by high performance liquid chromatography, by thin layer chromatography or by column chromatography using silica gel or magnesium silicate; or by washing or by recrystallization. Purification may be carried out after each reaction or after a series of reactions.

### Toxicity

The toxicity of the compound of the present invention represented by the formula (I) is very low and therefore, it is confirmed that the compound is sufficiently safe for use as medicine.

### Application to Pharmaceuticals

The compound of the present invention represented by the formula (I) binds to a CRF receptor to exhibit antagonist action, and therefore is useful for preventing and/or treating CRF mediated diseases, for example, neuropsychiatric diseases, digestive diseases, respiratory diseases, endocrine diseases, metabolic diseases, cardiovascular diseases, dermatologic diseases, genitourinary diseases, ophthalmologic diseases, or musculoskeletal diseases.

More specifically, as neuropsychiatric diseases, for example, mood disorders (e.g., depression, single episode depression, recurrent depression, postpartum depression, child abuse induced depression, bipolar disorder, and premenstrual dysphoric disorder); anxiety disorders (e.g. generalized anxiety disorder, panic disorder, obsessive compulsive disorder, social anxiety disorders, and phobic anxiety disorders (e.g. acrophobia, claustrophobia, agoraphobia, and social phobia)); stress-related disorders (e.g. posttraumatic stress disorder (PTSD), stress induced immunosuppression, stress induced headache, stress induced fever, stress induced pain, post operative stress, stress induced gastrointestinal disorder (e.g. gastritis, gastric ulcer, and duodenal ulcer, etc.), irritable bowel syndrome, hyposexuality, and erectile dysfunction)); eating disorders (e.g. anorexia nervosa, binge eating disorder, and nervous vomiting); symptom caused by psychotropic substance or dependency thereon (e.g. alcoholic withdrawal symptoms, alcohol dependence, drug addiction, and drug dependency); organic mental disorder (e.g. senile dementia of Alzheimer's type, and multi-infarct dementia); schizophrenic disorder; attention-deficit hyperactivity disorder; neurodegenerative diseases (e.g. Alzheimer's disease, Parkinson's disease, Huntington's disease, and amyotrophic lateral sclerosis); pain; convulsive disorders (e.g. convulsion and muscle spasm); episodic diseases (e.g. epilepsy, attack, and migraine); or sleep disorders (e.g. nonorganic sleep disorder, and fiber myalgic sleep disorder) are given. As digestive diseases, for example, peptic ulcer (e.g. gastric ulcer and duodenal ulcer); inflammatory bowel disease (e.g. ulcerative colitis and Crohn's disease); irritable bowel syndrome; stress induced gastrointestinal disorder (e.g. gastritis, gastric ulcer, and duodenal ulcer, etc.); diarrhea; and constipation are given. As respiratory diseases, for example, asthma, bronchitis, chronic obstructive pulmonary disease, or allergic rhinitis is given. As endocrine diseases, for example, disturbed thyroid function, Cushing's disease or syndrome of inappropriate antidiuretic hormone secretion is given. As metabolic diseases, for example, obesity or hypoglycemia is given. As cardiovascular diseases, for example, hypertension, ischemic heart disease, tachycardia, congestive heart failure, or cerebral vascular disease is given. As dermatologic diseases, for example, atopic dermatitis, allergic contact dermatitis or psoriasis is given. As genitourinary diseases, for example, urinary disturbance, pollakiuria or urinary incontinence is given. As ophthalmologic diseases, for example, uveitis is given. As musculoskeletal disorders, for example, chronic rheumatoid arthritis, osteoarthrosis, or osteoporosis is given.

The compound of the present invention represented by the formula (I) may be administered as a concomitant drug with other medicaments to accomplish the following purposes:
(1) to supplement and/or enhance the preventive and/or therapeutic effect of the present compound;
(2) to improve the kinetics and/or absorption and reduce the dose of the present compound; and/or
(3) to reduce the side effects of the present compound.

A combination of the compound represented by the formula (I) and other medicaments may be administered in the form of the formulations having those components incorporated in one preparation, or may be administered in separate preparations. In the case where those medicaments are administered in separate preparations, they may be administered simultaneously or at different times. Further, in the latter case, the compound of the present invention represented by the formula (I) may be administered before the other medicaments. Alternatively, the other medicaments may be administered before the compound of the present invention represented by the formula (I). The method for the administration of those medicaments are the same or different.

The diseases on which the preventive and/or therapeutic effect of the above combination preparations works are not specifically limited but may be those for which the preventive and/or therapeutic effect of the compound of the present invention represented by the formula (I) is supplemented and/or enhanced.

Examples of other medicaments for supplementing and/or enhancing the preventive and/or therapeutic effect of the compound of the present invention represented by the formula (I) on mood disorders include an antidepressant, a psychoanaleptic, an antianxiety agent, an antipsychotic agent, a mitochondrial benzodiazepine receptor (MBR) ligand, a neurokinin-1 (NK1) antagonist, and the like.

Examples of other medicaments for supplementing and/or enhancing the preventive and/or therapeutic effect of the compound of the present invention represented by the formula (I) on anxiety disorders include an antianxiety agent and a MBR ligand.

Examples of other medicaments for supplementing and/or enhancing the preventive and/or therapeutic effect of the compound of the present invention represented by the formula (I) on irritable bowel syndrome include a gastrointestinal promotility agent, a histamine H₂ receptor antagonist, a proton pump inhibitor, a 5-HT₃ antagonist a 5-HT₄ agonist, an anticholinergic agent, an antidiarrheal drug, a laxative agent, an autonomic modulating agent, an antidepressant, an antianxiety agent, an MBR ligand, and the like.

As an antidepressant, for example, a tricyclic antidepressant (e.g. amitriptyline hydrochloride, imipramine hydrochloride, clomipramine hydrochloride, dosulepin hydrochloride, nortriptyline hydrochloride, lofepramine hydrochloride, trimipramine maleate, amoxapine); a tetracyclic antidepressant (e.g. maprotiline hydrochloride, mianserin hydrochloride, setiptiline maleate); a monoamine oxidase (MAO) inhibitor (safrazine hydrochloride); serotonin and noradrenaline reuptake inhibitors (SNRI) (e.g. milnacipran hydrochloride, venlafaxine hydrochloride); a selective serotonin reuptake inhibitor (SSRI) (e.g. fluvoxamine maleate, paroxetine hydrochloride, fluoxetine hydrochloride, citalopram hydrochloride); and a serotonin reuptake inhibitor (e.g. trazodone hydrochloride) are given.

As an antianxiety agent, for example, a benzodiazepine anxiolytic (e.g. alprazolam, oxazepam, oxazolam, cloxazolam, clorazepate dipotassium, chlordiazepoxide, diazepam, tofisopam, triazolam, prazepam, fludiazepam, flutazolam, flutoprazepam, bromazepam, mexazolam, medazepam, ethyl loflazepate, lorazepam); a thienodiazepine anxiolytic (e.g. etizolam and clotiazepam); and a non-benzodiazepine anxiolytic (e.g. tandospirone citrate and hydroxylzine hydrochloride) are given.

As a psychoanaleptic, for example, methylphenidate hydrochloride and pemoline are given.

As an antipsychotic agent, for example, sulpiride, trazodone hydrochloride, and serotonin-dopamine antagonist (e.g. risperidone, perospirone hydrochloride hydrate, quetiapine fumarate, and olanzapine) are given.

As a gastrointestinal promotility agent, for example, trimebutine maleate and polycarbophil calcium are given.

As a histamine H₂ receptor antagonist, for example, cimetidine, ranitidine, famotidine, nizatidine, lafutidine, etc. are given.

As a proton pump inhibitor, for example, omeprazole, lansoprazole, rabeprazole, etc. are given.

As a 5-HT₃ antagonist, for example, alosetron is given.

As a 5-HT₄ agonist, for example, tegaserod, cisapride and mosapride citrate are given.

The mass ratio of the compound represented by the formula (I) and the other medicaments is not specifically limited.

Any combination of two or more kinds of other medicaments may be administered.

Further, the other medicaments for supplementing and/or enhancing the preventive and/or therapeutic effect of the compound represented by the formula (I) include not only those found so far but also those which will be found on the basis of the above-mentioned mechanism.

For the purpose described above, the compounds of the present invention represented by the formula (I), a non-toxic salt thereof, or a combination of the compounds represented by the formula (I) and other medicaments may be normally administered systemically or locally, usually by oral or parenteral administration.

The doses to be administered are determined depending upon, for example, ages, body weights, symptoms, the desired therapeutic effects, the route of administration, and the duration of the treatment. For the human adult, the dose per person is generally from 1 mg to 1,000 mg, by oral administration, from one to several times per day, and from 0.1 mg to 100 mg, by parenteral administration (preferably, nasal drops, eye drops, or ointments), from one to several times per day, or continuous administration for 1 to 24 hours per day from vein.

As described above, of course, the doses to be used depend upon various conditions. Therefore, there are cases in which doses lower than or greater than the ranges specified above may be used.

In the case where the compound of the present invention represented by the formula (I), or the combination agent of the compound represented by the formula (I) and the other medicaments is administrated, those are used as solid preparations for internal use and liquid preparations for internal use for oral administration as well as preparations for injections, external preparations, suppositories, eye drops, inhalations and the like for parenteral administration.

Examples of the solid preparations for internal use for oral administration include tablets, pills, capsules, powders, granules and the like. The capsules include hard capsules and soft capsules. Further, the tablets include sublingual tablet, oral patch and orally disintegrating tablet.

Such solid preparations for internal use is prepared by a formulation method commonly employed by using one or more active substances without modification, or a mixture of one or more active substances with an excipient (lactose, mannitol, glucose, microcrystalline cellulose, starch, etc.), a binder (hydroxypropylcellulose, polyvinylpyrrolidone, magnesium metasilicate aluminate, etc.), a disintegrating agent (calcium cellulose glycolate, etc.), a lubricant (magnesium stearate, etc.), a stabilizer, a solubilizing agent (glutamic acid, aspartic acid, etc.). Further, if necessary, it may be coated with a coating agent (sucrose, gelatin, hydroxypropylcellulose, hydroxypropylmethylcellulose phthalate, etc.). It may be coated with two or more layers. Moreover, capsules made of an absorbable material such as gelatin are involved in the scope thereof.

The liquid preparations for internal use for oral administration include pharmaceutically acceptable aqueous solutions, suspensions, emulsions, syrups, elixirs and the like. Such liquid preparations are prepared by dissolving, suspending or emulsifying one or more active substances in a diluent commonly employed (purified water, ethanol or a mixture thereof, etc.). Such liquid forms may also further contain humectants, suspending agents, emulsifying agents, sweetening agents, flavoring agents, aroma, preservatives, buffers, and the like.

The dose forms of the external preparations for parenteral administration include ointments, gels, creams, fomentations, patches, liniments, atomized agents, inhalations, sprays, eye drops, nasal drops, and the like. Such preparations contain one or more active substances and are prepared by a known method or a commonly employed formulation.

Atomized agents, inhalations, and sprays may contain, in addition to a diluent commonly employed, a stabilizer such as sodium bisulfite, and a buffer for imparting isotonicity such as an isotonic agent such as sodium chloride, sodium citrate or citric acid. Methods for producing a spray are described in detail in, for example, US Pat. No. 2,868,691 and US Pat. No. 3,095,355. In addition, an aerosol may be used in place of the spray.

The injections for parenteral administration include solutions, suspensions, emulsions and solid injections to be dissolved or suspended before use. The injection is used by dissolving, suspending or emulsifying one or more active substances in a solvent. The solvent includes, for example, distilled water for injection, physiological saline, vegetable oils, alcohols such as propylene glycol, polyethylene glycol and ethanol, and the combinations thereof. The injection may further contain a stabilizer, a dissolution aid (glutamic acid, aspartic acid, Polysorbate 80 (registered trademark), etc.), a suspending agent, an emulsifier, a soothing agent, a buffer, a preservative, and the like. The injection may be produced by sterilizing at the final step or employing an aseptic process. Alternatively, it is also possible that an aseptic solid product such as a freeze-dried product is produced and sterilized or dissolved in aseptic distilled water for injection or another solvent before use.

Other compositions for parenteral administration include suppositories for colorectal administration, pessaries for vaginal administration, and the like, which contain one or more active substances, and are formulated in accordance with common method.

### [Examples]

Hereinafter, the present invention is described in detail by way of examples, but not limited thereto.

Solvents given in parentheses concerning chromatographic separation and TLC each indicate the elution solvent or the developing solvent employed, and the ratio is expressed in ratio by volume.

Solvents given in parentheses concerning NMR each indicate the solvent employed in measurement.

The nomenclature used in the description of the present invention is based on ACD/Name (registered trademark) (version 6.00, manufactured by Advanced Chemistry Development Inc.).

### Example 1: 1-(2-chloro-4-methoxyphenyl)propane-1-one

Propionic chloride (12.6 mL) was dropped to aluminum chloride (19.2 g) in carbon tetrachloride suspension (100 mL) under ice-cold, followed by stirring for 15 minutes. 3-chloroanisole (17.1 g) in carbon tetrachloride solution (30 mL) was dropped to the obtained mixture while the inner temperature was kept at 5°C. The mixture was stirred under ice-cold for 20 minutes. The reaction mixture was poured into the diluted hydrochloric acid/ice and extracted with ethyl acetate. The extract was washed with diluted hydrochloric acid, a saturated sodium hydrocarbon solution, and a saturated salt solution, and concentrated under reduced pressure after drying with anhydrous magnesium sulfate. The obtained residue was purified by silica gel column chromatography (hexane:ethyl acetate=20:1→10:1), to thereby obtain a title compound (12.1 g) having the following physical properties. TLC: Rf 0.57 (hexane:ethyl acetate=4:1);
¹H-NMR (300MHz, CDCl₃): δ 7.58 (d, J=8.7 Hz, 1H), 6.93 (d, J=2.7Hz, 1H), 6.83 (dd, J=2.7, 8.7 Hz, 1H), 3.84 (s, 3H), 2.97 (q, J=7.5 Hz, 2H), 1.20 (t, J=7.5 Hz, 3H).

### Example 2: 2-bromo-1-(2-chloro-4-methoxyphenyl)propane-1-one

Tribromophenyl trimethylammonium (21.8 g) was added to the compound produced in Example 1 in tetrahydrofuran solution (THF; 120 mL) at room temperature, followed by stirring for 10 minutes. The insoluble substance was filtered with a glass filter and the filtrate was concentrated under reduced pressure. The obtained residue was purified by silica gel chromatography (hexane:ethyl acetate = 10:1), to thereby obtain a title compound (17.9 g) having the following physical properties.
TLC: Rf 0.63 (benzene:hexane:ethyl acetate=4:4:1);
¹H-NMR (300 MHz, CDCl₃) : δ 7.57 (d, J=8.4 Hz, 1H), 6.94 (d, J=2.7 Hz, 1H), 6.85 (dd, J=2.7, 8.4 Hz, 1H), 5.33 (q, J=6.6 Hz, 1H), 3.85 (s, 3H), 1.89 (d, J=6.6 Hz, 3H).

### Example 3: tert-butyl{5-[1-(1H-1,2,3-benzotriazol-1-yl)-1-ethylpropyl]-3-methylpyridin-2-yl}carbamate

Di-tert-butyl dicarbonate (11 g) was added to 5-[1-(1H-1, 2,3-benzotriazol-1-yl)1-ethylpropyl]-3-methylpyridin-2-amine (compound described in J. Chem. Soc, Perkin Trans 1 1995 (24), 3129)(7.46 g) in ethyl acetate solution (100 mL), followed by refluxing by heat for 1.5 hours. The reaction mixture was concentrated under reduced pressure. The obtained residue was purified by silica gel column chromatography (hexane:ethyl acetate=2:1→1:1), to thereby obtain a title compound (6.2 g) having the following physical properties.
TLC: Rf 0.54 (hexane:ethyl acetate=1:2);
¹H-NMR (300MHz, CDCl₃): δ 8.25 (d, J=2.1 Hz, 1H), 8.06 (dt, J=8.1, 0.9 Hz, 1H), 7.28 (m, 1H), 7.18 (m, 1H), 6.79 (m, 1H), 6.72 (s, 1H), 2.75 (m, 2H), 2.58 (m, 2H), 2.15 (s, 3H), 1.52 (s, 9H), 0.68 (t, J=7.2 Hz, 6H).

### Example 4: tert-butyl{5-[(1E)-1-ethyl-1-propen-1-yl]-3-methylpyridin-2-yl}carbamate

Potassium t-butoxide (4.02 g) was added to the compound (6.2 g) produced in Example 3 in dimethyl aceteamide solution (50 mL), followed by stirring at 70°C for 2.5 hours. The reaction mixture was cooled, and extracted with ethyl acetate added with water. The extract was washed with water and a saturated salt solution, and concentrated by reduced pressure after drying with anhydrous magnesium sulfate. As a result, a title compound (5.7 g) having the following physical properties was obtained.
TLC: Rf 0.50 (hexane:ethyl acetate=3:1);
¹H-NMR (300 MHz, CDCl₃) : δ 8.20 (d, J=2.1 Hz, 1H), 7.43 (m, 1H), 6.73 (s, 1H), 5.69 (q, J=6.9 Hz, 1H), 2.47 (q, J=7.5 Hz, 2H), 2.28 (s, 3H), 1.79 (d, J=6.9 Hz, 3H), 1.51 (s, 9H), 0.96 (t, J=7.5 Hz, 3H).

### Example 5: tert-butyl[5-(1-ethylpropyl)-3-methylpyridin-2-yl]carbamate

10% palladium carbon (500 mg) was added to the compound (5.7 g) produced in Example 4 in methanol solution (60 mL) under argon gas atmosphere. The obtained mixture was stirred for 1 hour under hydrogen atmosphere. The palladium carbon was removed by filtration with Celite (trade name), the filtrate was concentrated under reduced pressure. The obtained residue was purified by silica gel column chromatography (hexane:ethyl acetate=4:1), to thereby obtain a title compound (3.9 g) having the following physical properties.
TLC: Rf 0.58 (hexane:ethyl acetate=3:1);
¹H-NMR (300 MHz, CDCl₃): δ 8.02 (d, J=2.4 Hz, 1H), 7.27 (m, 1H), 6.73 (s, 1H), 2.30 (m, 1H), 2.28 (s, 3H), 1.77-1.61 (m, 2H), 1.60-1.43 (m, 1H), 0.77 (t, J=7.5 Hz, 6H).

### Example 6: 5-(1-ethylpropyl)-3-methylpyridin-2-amine

4 N hydrochloric acid in ethyl acetate solution (40 mL) was added to the compound (3.9 g) produced in Example 5, followed by stirring for 1 hour at room temperature. The reaction mixture was made to be base using ammonium solution under ice-cold, and extracted with ethyl acetate. The extract was washed with water and a saturated salt solution. The obtained extract was concentrated under reduced pressure after drying with anhydrous magnesium sulfate, to thereby obtain a title compound (2.6 g) having the following physical properties.
TLC: Rf 0.12 (hexane:ethyl acetate=2:1);
¹H-NMR (300 MHz, CDCl₃): δ 7.71 (d, J=2.1 Hz, 1H), 7.06 (m, 1H), 4.26 (s, 2H), 2.18 (m, 1H), 2.13 (s, 3H), 1.65 (m, 2H), 1.46 (m, 2H), 0.77 (t, J=7.2 Hz, 6H).

### Example 7: 2-(2-chloro-4-methoxyphenyl)-6-(1-ethylpropyl)-3,8-dimethylimidazo[1,2-a]pyridine

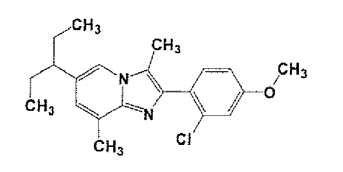

The compound (212 mg) produced in Example 2 and the compound (150 mg) produced in Example 6 in isopropyl alcohol (1.0 mL) were allowed to react for 15 minutes by microwave (200 W, 160°C). The reaction mixture was cooled and diluted with ethyl acetate. The extract was washed with water and a saturated salt solution, and concentrated under reduced pressure after drying with anhydrous magnesium sulfate. The obtained residue was purified by silica gel column chromatography (hexane:ethyl acetate=92:8→72:28), to thereby obtain a title compound (99 mg) as an oily substance having the following physical properties.
TLC: Rf 0.43 (hexane:ethyl acetate=3:1);
¹H-NMR (300 MHz, CDCl₃): δ 0.83 (t, J=7.3 Hz, 6H), 1.49-1.85 (m, 4H), 2.22-2.36 (m, 1H), 2.38 (s, 3H), 2.62 (s, 3H), 3.84 (s, 3H), 6.84 (s, 1H), 6.90 (dd, J=8.6, 2.6 Hz, 1H), 7.03 (d, J=2.6 Hz, 1H), 7.48 (d, J=8.6 Hz, 1H), 7.47-7.50 (m, 1H).

### Example 7 (1) -7 (11) :

The corresponding compounds were used, to thereby obtain the following compound by performing the same processes as in Example 1→Example 2→Example 3→Example 4→Example 5→Example 6→Example 7 in the stated order.

### Example 7(1): 6-(1-ethylpropyl)-2-(4-rnethoxy-2-methylphenyl)-3, 8-dimethylimidazo[1, 2-a]pyridine TLC: Rf 0.50 (hexane:ethyl acetate=2:1);

¹H-NMR (300 MHz, CDCl₃): δ 0.84 (t, J=7.3 Hz, 6H), 1.50-1.82 (m, 4H), 2.23-2.32 (m, 1H), 2.32 (s, 3H), 2.36 (s, 3H), 2.62 (s, 3H), 3.83 (s, 3H), 6.76-5.86 (m, 3H), 7.28 (d, J=8.2 Hz, 1H), 7.48 (s, 1H) .

### Example 7(2): 3-ethyl-6-(1-etrylpropyl)-2-(4-methoxy-2-methylphenyl)-8-methylimldazo[1,2-a]pyridine TLC: Rf 0.46 (hexane:ethyl acetate=3:1);

¹H-NMR (300 MHz, CDCl₃): δ 0.64 (t, J=7.5 Hz, 6H), 1.18 (t, J=7.5 Hz, 3H), 1.42-1.85 (m, 4H), 2.21-2.37 (m, 4H), 2.60 (s, 3H), 2.82 (q, J=7.5 Hz, 2H), 3.83 (s, 3H), 6.77 (dd, J=8.4, 2.4 Hz, 1H), 6.81 (s, 1H), 6.83 (d, J=2.4 Hz, 1H), 7.23 (d, J=8.4 Hz, 1H), 7.55 (s, 1H).

### Example 7(3): 2-(2-chloro-4-methoxyphenyl)-3-ethyl-6-(1-ethylpropyl)-8-methylimidazo[1,2-a]pyridine TLC: Rf 0.35 (hexane:ethyl acetate=3:1);

¹H-NMR (300 MHz, CDCl₃): δ 0.83 (t, J=7.3 Hz, 6H), 1.18 (t, J=7.5 Hz, 3H), 1.47-1.81 (m, 4H), 2.21-2.35 (m, 1H), 2.61 (s, 3H), 2.85 (q, J=7.5 Hz, 2H), 3.84 (s, 3H), 6.81 (s, 1H), 6.87 (dd, J=8.4, 2.6 Hz, 1H), 7.02 (d, J=2.6 Hz, 1H), 7.38 (d, J=8.6 Hz, 1H), 7.55 (s, 1H).

### Example 7(4): 2-(2-chloro-4-methoxyphenyl)-8-ethyl-6-(1-ethylpropyl)-3-methylimidazo[1, 2-a] pyridine TLC: Rf 0.44 (hexane:ethyl acetate=3:1);

¹H-NMR (300 MHz, CDCl₃) : δ 0.84 (t, J=7.4 Hz, 6H), 1.39 (t, J=7.5 Hz, 3H), 1.51-1.67 (m, 2H), 1.67-1.83 (m, 2H), 2.24-2.37 (m, 1H), 2.38 (s, 3H), 3.07 (q, J=7.5 Hz, 2H), 3.83 (s, 3H), 6.83 (d, J=1.3 Hz, 1H), 6.89 (dd, J=8.5, 2.7 Hz, 1H), 7.02 (d, J=2.6 Hz, 1H), 7.43-7.49 (m, 2H).

### Example 7(5): 2-(2-chloro-4-methoxyphenyl)-6-(1-ethylpropyl)-3-methylimidazo[1,2-a]pyridine TLC: Rf 0.46 (hexane:ethyl acetate=1:1);

¹H-NMR (300 MHz, CDCl₃) : δ 0.84 (t, J=7.3 Hz, 6H), 1.52-1.68 (m, 2H), 1.68-1.86 (m, 2H), 2.28-2.41 (m, 1H), 2.42 (s, 3H), 3.85 (s, 3H), 6.91 (dd, J=8.6, 2.6 Hz, 1H), 7.01-7.09 (m, 2H), 7.47 (d, J=8.6 Hz, 1H), 7.55-7.64 (m, 2H).

### Example 7(6): 2-(2-chloro-4-methoxyphenyl)-6-(1-ethylpropyl)imidazo[1,2-a]pyridine TLC: Rf 0.43 (hexane:ethyl acetate=2:1);

¹H-NMR (300 MHz, CDCl₃): δ 0.82 (t, J=7.3 Hz, 6H), 1.47-1.65 (m, 2H), 1.65-1.80 (m, 2H), 2.22-2.35 (m, 1H), 3.84 (s, 3H), 6.94 (dd, J=8.8, 2.7 Hz, 1H), 6.99-7.06 (m, 2H), 7.56 (d, J=9.3 Hz, 1H), 7.88 (s, 1H), 8.14 (s, 1H), 8.20 (d, J=8.8 Hz, 1H).

### Example 7(7): 2-(2-chloro-4-methoxyphenyl)-6-(1-ethylpropyl)-8-methylimidazo[1,2-a]pyridine

TLC: Rf 0.47 (toluene:ethyl acetate=10:1);
¹H-NMR (300 MHz, CDCl₃): δ 0.81 (t, J=7.4 Hz, 6H), 1.46-1.63 (m, 2H), 1.63-1.80 (m, 2H), 2.16-2.30 (m, 1H), 2.63 (s, 3H), 3.84 (s, 3H), 6.80 (s, 1H), 6.94 (dd, J=8.7, 2.7 Hz, 1H), 7.00 (d, J=2.4 Hz, 1H), 7.75 (s, 1H), 8.08 (s, 1H), 8.21 (d, J=8.8 Hz, 1H).

### Example 7(8): 2-(2-chloro-4-methoxyphenyl)-3-ethyl-6-(1-ethylpropyl)imidazo[1,2-a]pyridine TLC: Rf 0.27 (hexane:ethyl acetate=2:1);

¹H-NMR (300 MHz, CDCl₃): δ 0.84 (t, J=7.4 Hz, 6H), 1.21 (t, J=7.5 Hz, 3H), 1.50-1.68 (m, 2H), 1.68-1.87 (m, 2H), 2.27-2.42 (m, 1H), 2.89 (q, J=7.5 Hz, 2H), 3.85 (s, 3H), 6.89 (dd, J=8.5, 2.7 Hz, 1H), 6.98-7.07 (m, 2H), 7.39 (d, J=8.4 Hz, 1H), 7.58 (d, J=9.1 Hz, 1H), 7.68 (s, 1H).

### Example 7(9): 3-[2-(2-chloro-4-methoxyphenyl)-3,8-dimethylimidazo[1,2-a]pyridin-6-yl]-3-pentanol TLC: Rf 0.36 (hexane:ethyl acetate=1:1);

¹H-NMR (300 MHz, CDCl₃): δ 0.82 (t, J=7.4 Hz, 6H), 1.81-1.98 (m, 4H), 2.40 (s, 3H), 2.63 (s, 3H), 3.85 (s, 3H), 6.82 (s, 1H), 6.90 (dd, J=8.5, 2.7 Hz, 1H), 7.03 (d, J=2.4 Hz, 1H), 7.48 (d, J=8.6 Hz, 1H), 7.89 (s, 1H).

### Example 7(10): 2-(2-chloro-4-methoxyphenyl)-N,N-diethyl-3, 8-dimethylimidazo[1,2-a]pyridin-6-amine TLC: Rf 0.40 (hexane:ethyl acetate=1:2);

¹H-NMR (300 MHz, CDCl₃): δ 1.14 (t, J=7.0 Hz, 6H), 2.33 (s, 3H), 2.60 (s, 3H), 3.26 (q, J=7.1 Hz, 4H), 3.83 (s, 3H), 6.81-6.86 (m, J=2.2, 1.1 Hz, 1H), 6.88 (dd, J=8.6, 2.6 Hz, 1H), 7.00 (d, J=2.6 Hz, 1H), 7.05 (d, J=2.0 Hz, 1H), 7.46 (d, J=8.4 Hz, 1H).

### Example 7(11): 2-(2-chloro-4-methoxyphenyl)-N,N-dipropyl-3, 8-dimethylimidazo[1,2-a]pyridin-6-amine TLC: Rf 0.60 (hexane:ethyl acetate=1:2);

¹H-NMR (300 MHz, CDCl₃) : δ 0.94 (t, J=7.3 Hz, 6H), 1.50-1.68 (m, 4H), 2.32 (s, 3H), 2.60 (s, 3H), 3.10-3.21 (m, 4H), 3.83 (s, 3H), 6.81 (dd, J=2.2, 1.1 Hz, 1H), 6.88 (dd, J=8.6, 2.6 Hz, 1H), 6.99 (d, J=2.0 Hz, 1H), 7.00 (d, J=2.6 Hz, 1H), 7.46 (d, J=8.6 Hz, 1H).

### Example 8: Ethyl 3-(2-chloro-4-methoxyphenyl)-3-oxopropanoate

Zinc powders (7.83 g) in THF suspension (70 mL) was refluxed under heat. Bromo ethyl acetate (0.6 mL) was added to the suspension for 30 minutes under argon gas atmosphere, followed by stirring for 30 minutes. 2-chloro-4-methoxybenzonitrile (4.0 g) was added to the reaction mixture, and sequentially, bromo ethyl acetate (10 mL) was dropped, followed by stirring for 30 minutes. After the reaction mixture was cooled to room temperature, THF (200 mL) and 50% potassium carbonate (29 mL) were added thereto, and the mixture was stirred hard for 30 minutes. An organic layer was separated, and an insoluble substance was washed twice with THF. 2 N hydrochloric acid (24 mL) was added to the mixture solution of the organic layer and a washing solution, and then stirred for 2 hours. The reaction solution was concentrated under reduced pressure, and extracted with ethyl acetate. The extract was washed with a saturated salt solution, and concentrated under reduced pressure after drying with anhydrous magnesium sulfate. The obtained residue was purified by silica gel column chromatography (toluene:ethyl acetate=20:1), to thereby obtain a title compound (3.91 g) having the following physical properties.
TLC: Rf 0.56 (toluene:ethyl acetate=10:1);
¹H-NMR (300 MHz, CDCl₃): δ 7.73 (d, J=8.7 Hz, 1H), 6.94 (d, J=2.4 Hz, 1H), 6.86 (dd, J=2.4, 8.7 Hz, 1H), 4.20 (q, J=7.2 Hz, 2H), 4.03 (s, 2H), 3.86 (s, 3H), 1.25 (t, J=7.2 Hz, 3H).

### Example 9: Ethyl 2-bromo-3-(2-chloro-4-methoxyphenyl)-3-oxopropanoate

Ammonium acetate (54 mg) and N-bromosuccinimide (2.63 g) were added to the compound (3.61 g) produced in Example 8 in diethyl ether solution (30 mL) at room temperature, followed by stirring for 1 hour. The reaction mixture was diluted with water and extracted with ethyl acetate. The extract was washed with water and a saturated salt solution, and concentrated under reduced pressure after drying with anhydrous magnesium sulfate. The obtained residue was purified by silica gel column chromatography (hexane:ethyl acetate=89:11→68:32), to thereby obtain a title compound (3.84 g) having the following physical properties.
TLC: Rf 0.38 (hexane:ethyl acetate=3:1);
¹H-NMR (300 MHz, CDCl₃) : δ 7.66 (d, J=8.4 Hz, 1H), 6.95 (d, J=2.7 Hz, 1H), 6.86 (dd, J=2.7, 8.4 Hz, 1H), 5.82 (s, 1H), 4.27 (q, J=7.2 Hz, 2H), 3.86 (s, 3H), 1.27 (t, J=7.2 Hz, 3H).

### Example 10: Ethyl 2-(2-Chloro-4-methoxyphenyl)-6-(1-ethylpropyl)-8-methylimidazo[1,2-a]pyridine-3-carboxylate

A title compound having the following physical properties was obtained by using the compound produced in Example 6 and the compound produced in Example 9 and performing the same process as in Example 7.
TLC: Rf 0.36 (toluene:hexane:ethyl acetate=4:4:1);
¹H-NMR (300 MHz, CDCl₃): δ 0.83 (t, J=7.3 Hz, 6H), 1.08 (t, J=7.1 Hz, 3H), 1.51-1.68 (m, 2H), 1.69-1.85 (m, 2H), 2.32-2.45 (m, 1H), 2.66 (s, 3H), 3.85 (s, 3H), 4.20 (q, J=7.1 Hz, 2H), 6.89 (dd, J=8.5, 2.5 Hz, 1H), 7.01 (d, J=2.6 Hz, 1H), 7.07-7.11 (m, 1H), 7.40 (d, J=8.6 Hz, 1H), 9.03 (s, 1H).

### Example 10(1): Methyl 2-(2-chloro-4-methoxyphenyl)-6-(1-ethylpropyl)-8-methylimidazo[1,2-a]pyridine-3-carboxylate

A title compound having the following physical properties was obtained by using the compound produced in

### Example 6 and a corresponding compound and performing the same process as in Example 10.

TLC: Rf 0.54 (hexane:ethyl acetate=2:1);
¹H-NMR (300 MHz, CDCl₃): δ 0.83 (t, J=7.3 Hz, 6H), 1.50-1.86 (m, 4H), 2.28 - 2.51 (m, 1H), 2.66 (s, 3H), 3.74 (s, 3H), 3.86 (s, 3H), 6.90 (dd, J=8.6, 2.6 Hz, 1H), 7.02 (d, J=2.6 Hz, 1H), 7.10 (s, 1H), 7.42 (d, J=8.6 Hz, 1H), 9.01 (s, 1H).

### Example 11: 2-(2-chloro-4-methoxyphenyl)-6-(1-ethylpropyl)-8-methylimidazo[1,2-a]pyridine-3-carboxylate

2 N sodium hydroxide solution (1.6 mL) was added to the compound (454 mg) produced in Example 10 in ethanol solution (6 mL), followed by stirring at 75°C for 5 hours. 2 N hydrochloric acid (1.6 mL) was added to the reaction mixture cooled to room temperature, and the mixture was extracted with ethyl acetate. The extract was washed with water and a saturated salt solution, and concentrated under reduced pressure after drying with anhydrous magnesium sulfate. As a result, a title compound (421 mg) having the following physical properties was obtained.
TLC: Rf 0.25 (hexane:ethyl acetate=2:1);
¹H-NMR (300 MHz, CDCl₃) : δ 9.01 (s, 1H), 7.43 (d, J=8.4 Hz, 1H), 7.15 (s, 1H), 7.03 (d, J=2.7 Hz, 1H), 6.90 (dd, J=8.4, 2.7 Hz, 1H), 3.86 (s, 3H), 2.68 (s, 3H), 2.43 (m, 1H), 1.86-1.69 (m, 2H), 1.69-1.50 (m, 2H), 0.84 (t, J=7.2 Hz, 6H).

### Example 12: 2-(2-chloro-4-methoxyphenyl)-6-(1-ethylpropyl)-8-methylimidazo[1,2-a]pyridine-3-carboxamide

Oxalyl chloride (0.103 mL) was added to the compound (299 mg) produced in Example 11 in methylene chloride solution (3 mL) under argon gas atmosphere and under ice-cold, followed by stirring for 30 minutes. The reaction mixture was concentrated and diluted with THF. Ammonium solution was added to the diluted solution under ice-cold, and the solution was stirred for 10 minutes. The reaction mixture was extracted with ethyl acetate. The extract was washed with a saturated salt solution, and concentrated under reduced pressure after drying with anhydrous magnesium sulfate. The obtained residue was purified by silica gel column chromatography (hexane:ethyl acetate=71:29→50:50), to thereby obtain a compound (208 mg) having the following physical properties.
TLC: Rf 0.40 (hexane:ethyl acetate=1:1);
¹H-NMR (300 MHz, CDCl₃) : δ 0.83 (t, J=7.4 Hz, 6H), 1.51-1.68 (m, 2H), 1.68-1.84 (m, 2H), 2.35-2.46 (m, 1H), 2.64 (s, 3H), 3.87 (s, 3H), 5.32 (s, 2H), 6.96 (dd, J=8.6, 2.6 Hz, 1H), 7.06-7.09 (m, 1H), 7.09 (d, J=2.6 Hz, 1H), 7.46 (d, J=8.4 Hz, 1H), 9.26 (s, 1H).

### Example 12 (1) -12 (3) :

The same process as in Example 12 was carried out by using corresponding compounds to obtain the following compounds.

### Example 12(1): 2-(2-chloro-4-methoxyphenyl)-6-(1-ethylpropyl)-N, 8-dimethylimidazo[1,2-a]pyridine-3-carboxamide TLC: Rf 0.48 (hexane:ethyl acetate=1:1);

¹H-NMR (300 MHz, CDCl₃): δ 0.82 (t, J=7.3 Hz, 6H), 1.51-1.67 (m, 2H), 1.67-1.83 (m, 2H), 2.31-2.44 (m, 1H), 2.63 (s, 3H), 2.82 (d, J=4.8 Hz, 3H), 3.88 (s, 3H), 5.49 (d, J=4.8 Hz, 1H), 6.97 (dd, J=8.6, 2.6 Hz, 1H), 7.02-7.06 (m, 1H), 7.09 (d, J=2.6 Hz, 1H), 7.47 (d, J=8.6 Hz, 1H), 9.24 (s, 1H). Example 12(2): 2-(2-chloro-4-methoxyphenyl)-6-(1-ethylpropyl)-N,N, 8-trimethylimidazo[1,2-a]pyridine-3-carboxamide
TLC: Rf 0.48 (hexane:ethyl acetate=1:1);
¹H-NMR (300 MHz, CDCl₃): δ 0.82 (t, J=7.3 Hz, 6H), 1.48-1.65 (m, 2H), 1.65-1.82 (m, 2H), 2.25-2.37 (m, 1H), 2.42-3.17 (m, 9H), 3.85 (s, 3H), 6.90 (dd, J=8.6, 2.6 Hz, 1H), 6.94-6.98 (m, 1H), 7.03 (d, J=2.6 Hz, 1H), 7.50 (d, J=8.6 Hz, 1H), 8.27 (s, 1H).

### Example 12(3): 2-(2-chloro-4-methoxyphenyl)-6-(1-ethylpropyl)imidazo[1,2-a]pyridine-3-carboxamide TLC: Rf 0.33 (hexane:ethyl acetate=1:1);

¹H-NMR (300 MHz, CDCl₃): δ 0.84 (t, J=7.4 Hz, 6H), 1.53 - 1.68 (m, 2H), 1.70 - 1.84 (m, 2H), 2.40 - 2.50 (m, 1H), 3.87 (s, 3H), 5.34 (s, 2H), 6.96 (dd, J=8.6, 2.4 Hz, 1H), 7.09 (d, J=2.4 Hz, 1H), 7.28 (dd, J=9.2, 1.7 Hz, 1H), 7.45 (d, J=8.6 Hz, 1H), 7.64 (dd, J=9.2, 0.7 Hz, 1H), 9.34 - 9.41 (m, 1H).

### Example 13: 2-(2-chloro-4-methoxyphenyl)-6-(1-ethylpropyl)-8-methylimidazo[1,2-a]pyridine-3-carbonitrile

Phosphorous oxychloride (300 mg) was added to the compound (208 mg) produced in Example 12 in toluene solution (3.0 mL), followed by refluxing under heat for 3 hours. The reaction mixture was cooled to decompose extra phosphorous oxychloride using cold ice. The obtained mixture was made to be basic using a saturated sodium hydrocarbon solution, and was extracted with ethyl acetate. The extract was washed with water and a saturated salt solution, and concentrated under reduced pressure after drying with anhydrous magnesium sulfate. The obtained residue was purified by silica gel column chromatography (hexane:ethyl acetate=92:8→71:29), to thereby obtain a title compound (190 mg) having the following physical properties.
TLC: Rf 0.55 (hexane:ethyl acetate=2:1);
¹H-NMR (300 MHz, CDCl₃): δ 0.83 (t, J=7.4 Hz, 6H), 1.50-1.68 (m, 2H), 1.69 - 1.86 (m, 2H), 2.30 - 2.43 (m, 1H), 2.66 (s, 3H), 3.86 (s, 3H), 6.92 (dd, J=8.6, 2.6 Hz, 1H), 7.06 (d, J=2.6 Hz, 1H), 7.09 (s, 1H), 7.59 (d, J=8.6 Hz, 1H), 7.96 (s, 1H) .

### Example 14: Ethyl (2-amino-5-bromo-3-methylphenyl)carbamate

Sodium hydride (1.0 g) was added little by little to 5-bromo-3-methylbenzene-1,2-diamine (5.0 g) in dimethylformamide solution (85 mL) under argon gas atmosphere at 0°C, followed by stirring at 50°C for 1 hour. Ethyl chloroformate (2.4 mL) was dropped to the reaction solution cooled to 0°C, and the reaction solution was stirred at the same temperature for 30 minutes. The reaction solution added with a saturated ammonium chloride solution was poured into water, and was extracted with ethyl acetate. The extract was washed with water and a saturated salt solution, and concentrated under reduced pressure after drying with anhydrous magnesium sulfate. The obtained residue was purified by silica gel column chromatography (hexane:ethyl acetate=70:30→45:55), to thereby obtain a compound (3.8 g, isomer mixture at a ratio of 4:1) having the following physical properties.
TLC: Rf 0.43 (hexane:ethyl acetate=1:1).

### Example 15: 5-bromo-N¹,3-dimethylbenzene-1,2-diamine

Lithium aluminium hydride (1.5 g) was added little by little to the compound (3.6 g) produced in Example 14 in tetrahydrofuran solution (THF; 45 mL) at room temperature under argon gas atmosphere, followed by refluxing under heat for 1 hour. The reaction solution was cooled to 0°C and diluted with THF (45 mL). A saturated sodium sulfate solution was added to the diluted solution, and anhydrous magnesium sulfate was added thereto. The mixture solution was further stirred for 30 minutes. The mixture solution was filtered with Celite (trade name), the filtrate was concentrated under reduced pressure. The obtained residue was purified by silica gel column chromatography (hexane:ethyl acetate=90:10), to thereby obtain a title compound (1.8 g) having the following physical properties.
TLC: Rf 0.38 (chloroform:ethyl acetate=9:1);
¹H-NMR (300 MHz, CDCl₃): δ 6.74 (d, J=2.1 Hz, 1H), 6.65 (d, J=2.1 Hz, 1H), 3.26 (brs, 3H), 2.84 (s, 3H), 2.17 (s, 3H).

### Example 16: 6-bromo-2-(2-chloro-4-methoxyphenyl)-1,4-dimethyl-1H-benzimidazole

Activated carbon (Darco KB (trade name), manufactured by Aldrich Inc.); 100 mg) was added to the compound (200 mg) produced in Example 15 and 2-chloro-4-methoxybenzaldehyde (160 mg) in xylene solution (2 mL), followed by refluxing under heat for 12 hours under oxygen atmosphere. The reaction solution was cooled to room temperature, ethyl acetate was added thereto, and the reaction solution was subjected to filtration with Celite (trade name). The filtrate was concentrated under reduced pressure, whereby obtained solid was added with hexane, and filtrated to obtain a title compound (260 mg) having the following physical properties. TLC: Rf, 0.36 (toluene:ethyl acetate=3:1);
¹H-NMP (300 MHz, CDCl₃): δ 7.71 (m, 1H), 7.55 (d, J=8.7 Hz, 1H), 7.27 (d, J=2.4 Hz, 1H), 7.24 (m, 1H), 7.10 (dd, J=8.7, 2.4 Hz, 1H), 3.89 (s, 3H), 3.59 (s, 3H), 2.54 (s, 3H).

### Example 17: 2-(2-chloro-4-methoxyphenyl)-6-(1-ethylpropyl)-1,9-dimethyl-1H-benzimidazole

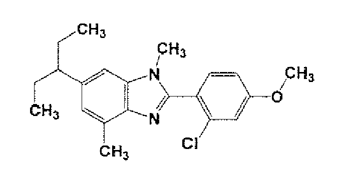

[1,1'-bis(diphenylphosphino)ferrocene]dichloropalladium (28 mg) and copper iodide (14 mg) were added to the compound (253 mg) produced in Example 16 in anhydrous THF (3.5 mL) at room temperature under argon gas atmosphere, whereby bromo(1-ethylpropyl)zinc (2.1 mL; 0.5 M THF solution) was further added thereto. The mixture was stirred at 50°C for 1.5 hours. The reaction solution was cooled to room temperature, and saturated ammonium chloride solution and hexane/ethyl acetate (1/1) were added thereto, and the reaction mixture was subjected to filtration with Celite (trade name). The filtrate was poured into water and extracted with ethyl acetate. The extract was washed with a saturated salt solution, and concentrated under reduced pressure after drying with anhydrous magnesium sulfate. The obtained residue was purified by silica gel column chromatography (hexane:ethyl acetate=75:25→54:46) to obtain a solid (225 mg). The solid was recrystallized with hexane/ethyl acetate, with the result that a title compound (95 mg) as colorless needle crystal having the following physical properties was obtained.
TLC: Rf 0.51 (hexane:ethyl acetate=1:1);
¹H-NMR (300 MHz, CDCl₃): δ 0.82 (t, J=7.3 Hz, 6H), 1.53-1.85 (m, 4H), 2.35-2.49 (m, 1H), 2.68 (s, 3H), 3.62 (s, 3H), 3.87 (s, 3H), 6.87-6.98 (m, 3H), 7.05 (d, J=2.4 Hz, 1H), 7.49 (d, J=8.6 Hz, 1H).

### Example 18: 4-(2-chlorc-4-methoxyphenyl)-2-methyl-3-butyn-2-ol

Tri-tert-butylphosphine (0.34 mL, 30% toluene solution) and diisopropylamine (3.5 mL) were added to dichlorobis(benzonitrile)palladium (260 mg) and copper iodide (86 mg) in dioxane solution (23 mL) at room temperature under argon gas atmosphere. Subsequently, 1-bromo-2-chloro-methoxybenzene (5.0 g) and 2-methyl-3-butyn-2-ol (2.6 mL) were slowly added thereto, followed by stirring at room temperature for 1 hour. Ethyl acetate (100 mL) was added to the reaction solution, and the reaction solution was subjected to filtration with Celite (trade name). After the filtrate was poured into water, the reaction solution was extracted with ethyl acetate. The extract was washed with a saturated salt solution, and concentrated under reduced pressure after drying with anhydrous magnesium sulfate. The obtained residue was purified by silica gel column chromatography (hexane:ethyl acetate=76:24→65:35), to thereby obtain a title compound (3.3 g) having the following physical properties.
TLC: Rf 0.55 (hexane:ethyl acetate=1:1);
¹H-NMR (300 MHz, CDCl₃): δ 7.35 (d, J=8.7 Hz, 1H), 6.93 (d, J=2.7 Hz, 1H), 6.75 (dd, J=8.7, 2.7 Hz, 1H), 3.80 (s, 3H), 3.71 (s, 1H), 1.64 (s, 6H).

### Example 19: 2-chloro-1-ethynyl-4-methoxybenzene

Sodium hydroxide powders (180 mg) were added to the compound (1.0 g) produced in Example 18 in toluene solution (20 mL) at room temperature, followed by refluxing under heat for 1 hour under argon gas atmosphere. The reaction solution was cooled and added with a saturated salt solution, and the reaction solution was extracted twice with toluene. The extract was concentrated under reduced pressure after drying with anhydrous magnesium sulfate. The residue was purified by silica gel column chromatography (hexane:ethyl acetate=95:5→74:26), to thereby obtain a title compound (585 mg) having the following physical properties.
TLC: Rf 0.59 (hexane:ethyl acetate=2:1);
¹H-NMR (300 MHz, CDCl₃): δ 7.42 (d, J=8. 7 Hz, 1H), 6.93 (d, J=2.4 Hz, 1H), 6.75 (dd, J=8.7, 2.4 Hz, 1H), 3.81 (s, 3H), 3.27 (s, 1H).

### Example 20: Ethyl 3-(2-chloro-4-methoxyphenyl)-2-propinoate

Ethylmagnesium bromide (1.4 mL, 3.0 M diethyl ether solution) was added to the compound produced in Example 19 (581 mg) in anhydrous tetrahydrofuran solution (10 mL) under argon gas atmosphere, followed by stirring for 1 hour at room temperature. Chloroethyl formate (0.4 mL) was added to the reaction solution at 0°C, and the resultant mixture was stirred at room temperature for 1 hour, thereafter being stirred at 50°C for 1 hour. After the reaction solution was cooled, ethanol was added thereto, whereby the resultant solution was poured into water and extracted with ethyl acetate. The extract was washed by a saturated salt solution, and concentrated under reduced pressure after drying with anhydrous magnesium sulfate. The obtained residue was purified by silica gel chromatography (hexane:ethyl acetate=99:1→75:25), to thereby obtain a compound (570 mg) having the following physical properties.
TLC: Rf 0.50 (hexane:ethyl acetate=2:1);
¹H-NMR (300 MHz, CDCl₃) : δ 7.53 (d, J=9.0 Hz, 1H), 6.97 (d, J=2.4 Hz, 1H), 6.80 (dd, J=9.0, 2.4Hz, 1H), 4.30 (q, J=7.2 Hz, 2H), 3.84 (s, 3H), 1.36 (t, J=7.2 Hz, 3H).

### Example 21: 4-(1-ethylpropyl)-2-methylpyridine

[1,1'-bis(diphenylphosphinoferrocene)]dichloropalladium (800 mg) and copper iodide (373 mg) were added to 4-chloro-2-methylpyridine (2.5 g) in an anhydrous tetrahydrofuran solution (10 mL) under argon gas atmosphere. 1-ethyl-n-propylmagnesium bromide (47 mL, 0.5 M tetrahydrofuran solution) was further added thereto, and the reaction solution was subjected to reflux under heat for 3 hours. After cooled to room temperature, the reaction solution was separated by adding 1 N hydrochloric acid solution, hexane, and ethyl acetate. An organic layer was washed twice with 2 N hydrochloric acid. The organic layer and a water layer were combined and made to be alkaline using 5 N sodium hydroxide solution, followed by extraction with ethyl acetate. The organic layer was concentrated under reduced pressure after drying with anhydrous magnesium sulfate. The obtained residue was distilled off under reduced pressure (7 mmHg, fraction obtained at 49-50°C), to thereby obtain a title compound (650 mg) having the following physical properties.
TLC: Rf 0.61 (dichloromethane:methanol=9:1);
¹H-NMR (300 MHz, CDCl₃) : δ 8.37 (d, J=8.1 Hz, 1H), 6.92 (s, 1H), 6.87 (m, 1H), 2.53 (s, 3H), 2.27 (m, 1H), 1.77-1.44 (m, 4H), 0.77 (t, J=7.5 Hz, 6H).

### Example 22: 1-amino-4-(1-ethylpropyl)-2-methylpyridinium 2, 4-dinitrophenolate

o-(2, 4-dinitrophenyl)hydroxylamine (268 mg; referring to T. Sheradsky, J. Heterocycl. Chem. 4, 413 (1967)) was added to the compound (200 mg) produced in Example 21 in acetonitrile solution (4 mL), followed by stirring at 40°C under argon gas atmosphere for 5 hours. After concentrated under reduced pressure, the reaction solution was washed by diethyl ether, whereby diethyl ether was removed by decantation. After that, the resultant solution was concentrated under reduced pressure to obtain a title compound (475 mg) having the following physical properties.
TLC: Rf 0.36(dichloromethane:methanol=9:1);
¹H-NMR (300 MHz, CDCl₃) : δ 9.20 (d, J=6.6 Hz, 1H), 8.89 (d, J=3.0 Hz, 1H), 8.20 (brs, 2H), 8.00 (dd, J=9.6, 3.0 Hz, 1H), 7.40 (dd, J=6.6, 2.1 Hz, 1H), 7.36 (d, J=2.1 Hz, 1H), 6.64 (d, J=9.6 Hz, 1H), 2.79 (s, 3H), 2.53 (m, 1H), 1.93-1.70 (m, 2H), 1.67-1.48 (m, 2H), 0.81 (t, J=7.2 Hz, 6H).

### Example 23: Ethyl 2-(2-chloro-4-methoxyphenyl)-5-(1-ethylpropyl)-7-methylpyrazolo[1,5-a]pyridine-3-carboxylate

Potassium carbonate (170 mg) was added to the compound (475 mg) produced in Example 22 and the compound (196 mg) produced in Example 20 in dimethylsulfoxide (2.7 mL) under argon gas atmosphere, followed by stirring at room temperature for 5 hours. Water, ethyl acetate, and hexane were added to the reaction solution, and the resultant solution was poured into water, and the reaction solution was extracted with ethyl acetate. The extract was washed with water and a saturated salt solution sequentially, and concentrated under reduced pressure after drying with anhydrous magnesium sulfate. The obtained residue was purified by silica gel chromatography (hexane:ethyl acetate=100:0→86:14), to thereby obtain a title compound (210 mg) having the following physical properties. TLC: Rf 0.59 (hexane:ethyl acetate=2:1);
¹H-NMR (300 MHz, CDCl₃): δ 7.87 (d, J=1.5 Hz, 1H), 7.36 (d, J=8.4 Hz, 1H), 7.02 (d, J = 2 . Hz, 1H), 6.88 (dd, J=8.4, 2.4 Hz, 1H), 6.64 (d, J=1.5 Hz, 1H), 4.20 (q, J=7.2 Hz, 2H), 3.85 (s, 3H), 2.78 (s, 3H), 2.45 (m, 1H), 1.84-1.52 (m, 4H), 1.16 (t, J=6.9 Hz, 3H), 0.84 (t, J=7.2 Hz, 6H).

### Example 24: 2-(2-chloro-4-methoxyphenyl)-5-(1-ethylpropyl)-7-methylpyrazolo[1,5-a]pyridine-3-carboxylate

A title compound (75 mg) having the following physical properties was obtained by using the compound (100 mg) produced in Example 23 and performing the same process as in Example 11.
TLC: Rf 0.33 (hexane:ethyl acetate=2:1);
¹H-NMR (300 MHz, CDCl₃) : δ 7.92 (d, J=1.5 Hz, 1H), 7.41 (d, J=8.4 Hz, 1H), 7.06 (d, J=2.4 Hz, 1H), 6.91 (dd, J=8.4, 2.4 Hz, 1H), 6.68 (s, 1H), 3.87 (s, 3H), 2.79 (s, 3H), 2.49 (m, 1H), 1.86-1.52 (m, 4H), 0.84 (t, J=7.5 Hz, 6H).

### Example 25: 2-(2-chloro-4-methoxyphenyl)-5-(1-ethylpropyl)-7-methylpyrazolo[1,5-a]pyridine-3-carboxamide

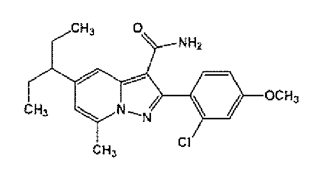

A title compound (57 mg) having the following physical properties was obtained by using the compound (75 mg) produced in Example 24 and performing the same process as in Example 12.
TLC: Rf 0.35 (hexane:ethyl acetate=1:2);
¹H-NMR (300 MHz, CDCl₃): δ 0.83 (t, J=7.3 Hz, 6 H), 1.50-1.84 (m, 4 H), 2.38 - 2.55 (m, 1 H), 2.77 (s, 3 H), 3.88 (s, 3 H), 5.19 (s, 2 H), 6.66 (d, J=1.5 Hz, 1 H), 6.97 (dd, J=8.6, 2.6 Hz, 1 H), 7.11 (d, J=2.6 Hz, 1 H), 7.45 (d, J=8.6 Hz, 1 H), 8.11 (d, J=1.5 Hz, 1 H).

### Example 26: 2-(2-chloro-4-methoxyphenyl)-5-(1-ethylpropyl)-7-methylpyrazolo[1,5-a]pyridine

47% hydrobromic acid (1 mL) was added to the compound (105 mg) produced in Example 23 under argon gas atmosphere, followed by stirring at 120°C for 2 hours. After cooled to room temperature, the reaction solution was neutralized using 5 N sodium hydroxide solution. After that, the resultant solution was poured into water, and was extracted with ethyl acetate. The extract was washed with a saturated salt solution, and concentrated under reduced pressure after drying with anhydrous magnesium sulfate. The obtained residue was purified by silica gel chromatography (hexane:ethyl acetate=100:0→70:30), to thereby obtain a title compound (19 mg) having the following physical properties and 3-chloro-4-[5-(1-ethylpropyl)-7-methylpyrazolo[1,5-a]pyridin-2-yl]phenol (33 mg).

Potassium carbonate (20 mg) and methyl iodide (10 µL) were added to 3-chloro-4-[5-(1-ethylpropyl)-7-methylpyrazolo[1,5-a]pyridin-2-yl]phenol (32 mg) in dimethylformamide solution (1 mL) under argon gas atmosphere, followed by stirring at 80°C for 2 hours. After cooled to room temperature, the reaction solution was poured into water, and was extracted with ethyl acetate. The extract was washed with a saturated salt solution, and concentrated under reduced pressure after drying with anhydrous magnesium sulfate. The obtained residue was purified by silica gel column chromatography (hexane:ethyl acetate=100:0→81:19), to thereby obtain a title compound (37 mg) having the following physical properties.
TLC: Rf 0.65 (hexane:ethyl acetate=2:1);
¹H-NMR (300 MHz, CDCl₃): δ 7.88 (d, J=8.7 Hz, 1H), 7.19 (s, 1H), 7.03 (d, J=2.7 Hz, 1H), 6.91 (dd, J=8.7, 2.7 Hz, 1H), 6.88 (s, 1H), 6.44 (s, 1H), 3.85 (s, 3H), 2.76 (s, 3H), 2.33 (m, 1H), 1.80-1.47 (m, 4H), 0.81 (t, J=7.2 Hz, 6H).

### Example 27: 3-bromo-2-(2-chloro-4-methoxyphenyl)-5-(1-ethylpropyl)-7-methylpyrazolo[1,5-a]pyridine

N-bromosuccinimide (29 mL) was added to the compound (55 mg) produced in Example 26 in acetonitrile solution (2 mL) under argon gas atmosphere, followed by stirring at room temperature for 1 hour. Ethyl acetate was added to the reaction solution, whereby the reaction solution was poured into water and extracted with ethyl acetate. The extract was washed with water and a saturated salt solution sequentially, and concentrated under reduced pressure after drying with anhydrous magnesium sulfate. The obtained residue was purified by silica gel column chromatography (hexane:ethyl acetate=100:0→83:17), to thereby obtain a title compound (65 mg) having the following physical properties.
TLC: Rf 0.43 (hexane:acetone=5:1);
¹H-NMR (380 MHz, CDCl₃): δ 7.43 (d, J=8.4 Hz, 1H), 7.17 (d, J=1.5 Hz, 1H), 7.07 (d, J=2.4 Hz, 1H), 6.91 (dd, J = 8.4, 2.4 Hz, 1H), 6.53 (d, J=1.5 Hz, 1H), 3.86 (s, 3H), 2.40 (m, 1H), 2.05 (s, 3H), 1.84-1.50 (m, 4H), 0.84 (t, J=7.5 Hz, 6H).

### Example 28: 2-(2-chloro-4-methoxyphenyl)-5-(1-ethylpropyl)-3, 7-dimethylpyrazolo[1,5-a]pyridine

Water (0.075 mL), methyl boronate (14 mg), palladium acetate (2 mg), tricyclohexylphosphine (15 µL; 30% toluene solution), and tripotassium phosphate (115 mg) were added to the compound (64 mg) produced in Example 27 in toluene solution (1.5 mL) at room temperature under argon gas atmosphere. After being degassed, the mixture was stirred at 100°C for 12 hours. After the reaction solution was cooled to room temperature, water and ethyl acetate were added thereto, whereby the reaction solution was filtered with Celite (trade name). The filtrate was poured into water, and the reaction solution was extracted with ethyl acetate. The extract was washed with water and a saturated salt solution sequentially, and concentrated under reduced pressure after drying with anhydrous magnesium sulfate. The obtained residue was purified by silica gel chromatography (hexane:ethyl acetate=99:1→78:22), to thereby obtain a title compound (35 mg) having the following physical properties.
TLC: Rf 0.34 (hexane:ethyl acetate=5:1);
¹H-NMR (300 MHz, CDCl₃): δ 0.83 (t, J=7.4 Hz, 6H), 1.48 - 1.81 (m, 4H), 2.20 (s, 3H), 2.27 - 2.43 (m, 1H), 2.73 (s, 3H), 3.85 (s, 3H), 6.42 (d, J=1.3 Hz, 1H), 6.90 (dd, J=8.4, 2.6 Hz, 1H), 7.05 (d, J=2.6 Hz, 1H), 7.07 (d, J=1.3 Hz, 1H), 7.40 (d, J=8.4 Hz, 1H).

### Example 29: 6-(1-ethylpropyl)-2-(4-methoxy-2-methylphenyl)-1,4-dimethyl-1H-benzimidazole

A title compound having the following physical properties was obtained by using the compound produced in Example 15 and 2-methyl-4-methoxybenzaldehyde in place of 2-chloro-4-methoxybenzaldehyde and performing the same processes as in Example 16→Example 17 in the stated order.
TLC: Rf 0.58 (hexane:ethyl acetate=1:1);
¹H-NMR (300 MHz, CDCl₃): δ 0.83 (t, J=7.3 Hz, 6 H), 1.52 - 1.85 (m, 4 H), 2.25 (s, 3H), 2.43 (m, 1 H), 2.68 (s, 3 H), 3.57 (s, 3 H), 3.85 (s, 3 H), 6.77 - 6.89 (m, 2 H), 6.91 (s, 1 H), 6.95 (s, 1 H), 7.31 (d, J=8.2 Hz, 1 H).

### Example 30: 6-(1-ethylpropyl)-2-(4-methoxy-2-methylphenyl)-8-methylimidazo[1,2-a]pyridine-3-carboxamide

A title compound having the following physical properties was obtained by using the compound produced in Example 6 and ethyl 2-bromo-3-(2-methyl-4-methoxyphenyl)-3-oxopropanoate in place of the compound produced in Example 9 and performing the same processes as in Example 10→Example 11→Example 12 in the stated order.
TLC: Rf 0.45 (hexane:ethyl acetate=1:1);
¹H-NMR (300 MHz, CDCl₃) : δ 0.83 (t, J=7.4 Hz, 6H), 1.49 - 1.85 (m, 4H), 2.24 (s, 3H), 2.33 - 2.49 (m, 1H), 2.59 - 2.68 (m, 3 H), 3.85 (s, 3H), 5.04 - 5.66 (m, 2 H), 6.84 - 6.91 (m, 2H), 7.05 - 7.10 (m, 1H), 7.34 (d, J=8.2 Hz, 1H), 9.32 (m, J=1.1, 0.4 Hz, 1H).

### Example 30(1)-30(14):

The following compounds were obtained by using corresponding compounds and performing the same process as in Example 30.

### Example 30(1): 2-(2, 4-dimethylphenyl)-6-(1-ethylpropyl)-8-methylimidazo[1,2-a]pyridine-3-carboxamide

TLC: Rf 0.37(hexane:ethyl acetate=1:1);
¹H-NMR (300 MHz, CDCl₃) : δ 0.84 (t, J=7.4 Hz, 6H), 1.49-1.86 (m, 4H), 2.21 (s, 3H), 2.42 (m, 1H), 2.38 (s, 3H), 2.64 (s, 3H), 5.09 (s, 1H), 5.47 (s, 1H), 7.08 (s, 1H), 7.14 (d, 2 H), 7.30 (d, J=7.5 Hz, 1H), 9.32 (s, 1H).

### Example 30(2): 6-(1-ethylpropyl)-8-methyl-2-[4-methyl-2-(trifluoromethyl)phenyl]imidazo[1,2-a]pyridine-3-carboxamide

TLC: Rf 0.44 (hexane:ethyl acetate=1:1);
¹H-NMR (300 MHz, CDCl₃) δ 0.84 (t, J=7.4 Hz, 6H), 1.49-1.87 (m, 4H), 2.41 (m, 1H), 2.50 (s, 3H), 2.63 (s, 3H), 5.08 (s, 2 H), 7.09 (s, 1H), 7.40 - 7.54 (m, 2H), 7.67 (s, 1H), 9.26 (s, 1H).

### Example 30(3): 6-(1-ethylpropyl)-8-methyl-2-(2, 4, 5-trimethylphenyl)imidazo[1,2-a]pyridine-3-carboxamide

TLC: Rf 0.49 (hexane:ethyl acetate=2:1);
¹H-NMR (300 MHz, CDCl₃): δ 0.83 (t, J=7.3 Hz, 6H), 1.52-1.67 (m, 2H), 1.69 - 1.82 (m, 2H), 2.17 (s, 3H), 2.25 (s, 3H), 2.28 (s, 3H), 2.60 (m, 1H), 2.63 (s, 3H), 5.10 (s, 1H), 5.54 (s, 1H), 7.06 (s, 1H), 7.09 (s, 1H), 7.16 (s, 1H), 9.30 (s, 1H).

### Example 30(4): 6-(1-ethylpropyl)-8-methyl-2-[2-methyl-4-(methylthio)phenyl]imidazo[1,2-a]pyridine-3-carboxamide

TLC: Rf 0.30 (hexane:ethyl acetate=2:1);
¹H-NMR (300 MHz, CDCl₃): δ 0.83 (t, J=7.4 Hz, 6 H), 1.50 - 1.85 (m, 4H), 2.24 (s, 3H), 2.41 (m, 1H), 2.52 (s, 3H), 2.64 (s, 3H), 5.12 (s, 1H), 5.44 (s, 1H), 7.08 (s, 1H), 7.16 - 7.25 (m, 2H), 7.34 (d, J=7.9 Hz, 1H), 9.32 (s, 1H).

### Example 30(5): 2-(2-ethyl-4-methoxyphenyl)-6-(1-ethylpropyl)-8-methylimidazo[1,2-a]pyridine-3-carboxamide

TLC: Rf 0.33 (hexane:ethyl acetate=2:1);
¹H-NMR (300 MHz, CDCl₃): δ 0.84 (t, J=7.3 Hz, 6H), 1.13 (t, J=7.6 Hz, 3H), 1.51-1.86 (m, 4H), 2.33-2.48 (m, 1H), 2.56 (q, J=7.6 Hz, 2H), 2.64 (s, 3H), 3.86 (s, 3H), 5.17 (m, 1H), 5.47 (m, 1H), 6.86 (dd, J=8.4, 2.6 Hz, 1H), 6.94 (d, J=2.4 Hz, 1H), 7.09 (m, J=1.1 Hz, 1H), 7.32 (d, J=8.4 Hz, 1H), 9.33 (s, 1H).

### Example 30(6): 2-(4-ethoxy-2-ethylphenyl)-6-(1-ethylpropyl)-8-methylimidazo[1,2-a]pyridine-3-carboxamide

TLC: Rf 0.38 (hexane:ethyl acetate=2:1);
¹H-NMR (300 MHz, CDCl₃) δ 0.84 (t, J=7.4 Hz, 6H), 1.12 (t, J=7.6 Hz, 3H), 1.45 (t, J=7.0 Hz, 3H), 1.51-1.84 (m, 4H), 2.33-2.48 (m, 1H), 2.55 (q, J=7.5 Hz, 2H), 2.64 (s, 3H), 4.09 (q, J=7.0 Hz, 2H), 5.15 (m, 1H), 5.49 (m, 1H), 6.84 (dd, J=8.3, 2.7 Hz, 1H), 6.94 (d, J=2.6 Hz, 1H), 7.08 (m, 1H), 7.30 (d, J=8.4 Hz, 1H), 9.33 (m, J=0.9 Hz, 1H).

### Example 30(7): 2-(2-chloro-4-methoxy-5-methylphenyl)-6-(1-ethylpropyl)-8-methylimidazo[1,2-a]pyridine-3-carboxamide TLC: Rf 0.56 (hexane:ethyl acetate=1:1);

¹H-NMR (300 MHz, CDCl₃) : δ 0.83 (t, J=7.3 Hz, 6H), 1.50-1.86 (m, 4H), 2.22 (s, 3H), 2.40 (m, 1H), 2.64 (s, 3H), 3.89 (s, 3H), 5.21 - 5.50 (m, 2H), 6.97 (s, 1H), 7.08 (m, 1H), 7.31 (d, J=0.7 Hz, 1H), 9.26 (s, 1H).

### Example 30(8): 6-(1-ethylpropyl)-8-methyl-2-(6-methyl-1,3-benzodioxol-5-yl)imidazo[1,2-a]pyridine-3-carboxamide

TLC: Rf 0.46 (hexane:ethyl acetate=1:1);
¹H-NMR (300 MHz, CDCl₃): δ 0.83 (t, J=7.4 Hz, 6H), 1.50-1.86 (m, 4H), 2.16 (s, 3H), 2.41 (m, 1 H), 2.63 (s, 3H), 5.18 (m, 1H), 5.46-5.69 (m, 1H), 6.00 (s, 2H), 6.82 (s, 1H), 6.88 (s, 1H), 7.08 (s, 1H), 9.31 (s, 1H).

### Example 30(9): 6-(1-ethylpropyl)-8-methyl-2-[4-(methylthio)-2-(trifluoromethyl)phenyl]imidazo[1,2-a]pyridine-3-carboxamide

TLC: Rf 0.42 (hexane:ethyl acetate=1:1);
¹H-NMR (300 MHz, CDCl₃): δ 0.84 (t, J=7.4 Hz, 6H), 1.50-1.87 (m, 4H), 2.39 (m, 1H), 2.57 (s, 3H), 2.62 (s, 3H), 5.12 (s, 2H), 7.10 (m, 1H), 7.43-7.54 (m, 2H), 7.68 (d, J=1.8 Hz, 1 H), 9.24 (m, 1H).

### Example 30(10): 6-(1-ethylpropyl)-2-[2-methoxy-4-(methylthio)phenyl]-8-methylimidazo[1,2-a]pyridine-3-carboxamide

TLC: Rf 0.31 (hexane:ethyl acetate=1:1);
¹H-NMR (300 MHz, CDCl₃): δ 0.82 (t, J=7.3 Hz, 6H), 1.47-1.85 (m, 4H), 2.38 (m, 1H), 2.54 (s, 3H), 2.63 (s, 3H), 3.81 (s, 3H), 5.43 (s, 2H), 6.92 (d, J=1.7 Hz, 1H), 6.97 (dd, J=7.9, 1.7 Hz, 1H), 7.04 (s, 1H), 7.41 (d, J=7.9 Hz, 1H), 9.19 (s, 1H).

### Example 30(11): 2-[6-(dimethylamino)-4-methyl-3-pyridinyl]-6-(1-ethylpropyl)-8-methylimidazo[1,2-a]pyridine-3-carboxamide

TLC: Rf 0.37 (ethyl acetate);
¹H-NMR (300 MHz, CBCl₃): δ 0.83 (t, J=7.3 Hz, 6H), 1.52 - 1.67 (m, 2H), 1.69 - 1.83 (m, 2H), 2.21 (s, 3H), 2.41 (m, 1H), 2.64 (s, 3H), 3.14 (s, 6H), 5.25 (m, 1H), 5.63 (m, 1H), 6.47 (s, 1H), 7.07 (s, 1H), 8.19 (s, 1H), 9.31 (s, 1H) .

### Example 30(12): 2-(2-ethyl-4, 5-dimethoxyphenyl)-6-(1-ethylpropyl)-8-methylimidaza[1,2-a]Pyridine-3-carboxamide

TLC: Rf 0.27 (hexane:ethyl acetate=1:1);
¹H-NMR (300 MHz, CBCl₃) : δ 0.84 (t, J=7.3 Hz, 6H), 1.12 (t, J=7.7 Hz, 3H), 1.48 - 1.87 (m, 4H), 2.41 (m, 1H), 2.53 (q, J=7.7 Hz, 2H), 2.64 (s, 3H), 3.85 (s, 3H), 3.95 (s, 3H), 5.10 (s, 1H), 5.54 (s, 1H), 6.83-6.93 (m, J=1.8 Hz, 2H), 7.09 (s, 1H), 9.33 (s, 1H).

### Example 30(13): 2-(4-ethoxy-2-methylphenyl)-6-(1-ethylpropyl)-8-methylimidazo[1,2-a]pyridine-3-carboxamide

TLC: Rf 0.52 (hexane:ethyl acetate=1:1);
¹H-NMR (300 MHz, CDCl₃): δ 0.83 (t, J=7.4 Hz, 6H), 1.44 (t, J=6.9 Hz, 3H), 1.55-1.67 (m, 2H), 1.69 - 1.80 (m, 2H), 2.22 (s, 3H), 2.41 (m, 1H), 2.64 (s, 3H), 4.08 (q, J=6.9 Hz, 2H), 5.08 (m, 1H), 5.50 (m, 1H), 6.83-6.89 (m, 2H), 7.07 (s, 1H), 7.32 (d, J=8.2 Hz, 1H), 9.32 (s, 1H).

### Example 30(14): 6-(1-ethylpropyl)-2-(4-methoxy-2,5-dimethylphenyl)-8-methylimidazo[1,2-a]pyridine-3-carboxamide

TLC: Rf 0.55 (hexane:ethyl acetate-1:1);
¹H-NMR (300 MHz, CDCl₃) : δ 0.83 (t, J=7.3 Hz, 6H), 1.55-1.68 (m, 2H), 1.69-1.83 (m, 2H), 2.21 (s, 3H), 2.22 (s, 3H), 2.40 (m, 1H), 2.64 (s, 3H), 3.87 (s, 3H), 5.04 (m, 1H), 5.58 (m, 1H), 6.78 (s, 1H), 7.07 (s, 1H), 7.17 (s, 1H), 9.32 (s, 1H) .

### Example 31: Ethyl 2-(2-chloro-4-methoxyphenyl)-3-methyl-6-(1-ethylpropyl)imidazo[1,2-a]pyridine-8-carboxylate

To 2-(2-chloro-4-methoxyphenyl)-3-methyl-6-(1-ethylpropyl)-8-bromoimidazo[1,2-a]pyridine (259 mg) in dimethylsulfoxide solution (0.6 mL) obtained by using the compound produced in Example 2 and 5-(1-ethylpropyl)-3-bromopyridin-2-amine in place of the compound produced in Example 6 and performing the same process as in Example 7, added were ethanol (0.6 mL), palladium acetate (14 mg), 1,1'-bis(diphenylphosphino)ferrocene (34 mg), diisopropylethylamine (0.26 mL), followed by stirring at 70°C for 4 hours under carbon monoxide atmosphere. Water was added to the reaction solution, and the extract extracted with ethyl acetate was washed with water and a saturated salt solution. The resultant solution was concentrated under reduced pressure after drying with anhydrous magnesium sulfate. The obtained residue was purified by silica gel column chromatography (hexane:ethyl acetate=70:30→50:50), to thereby obtain a title compound (140 mg) having the following physical properties. TLC: Rf 0.50 (hexane:ethyl acetate=1:1);
¹H-NMR (300 MHz, CDCl₃) : δ 0.84 (t, J=7.4 Hz, 6H), 1. 44 (t, J=7.1 Hz, 3H), 1.62-1.70 (m, 2H), 1.73-1.86 (m, 2H), 2.39 (m, 1H), 2.43 (s, 3H), 3.85 (s, 3H), 4.49 (q, J=7.1 Hz, 2H), 6.90 (dd, J=8.4, 2.6 Hz, 1H), 7.00 (d, J=2.6 Hz, 1H), 7.55 (d, J=8.4 Hz, 1H), 7.75-7.76 (m, 2H).

### Example 32: 2-(2-chloro-4-methoxyphenyl)-6-(1-ethylpropyl)-3-methylimidazo[1,2-a]pyridine-8-carbonitrile

A title compound (7 mg) having the following physical properties was obtained by using the compound (33 mg) produced in Example 31 and performing the same processes as in Example 11→ Example 12→Example 13 in the stated order.
TLC: Rf 0.43 (hexane:ethyl acetate=2:1);
¹H-NMR (300 MHz, CDCl₃): δ 0.85 (t, J=7.3 Hz, 6H), 1.52-1.66 (m, 2H), 1.73-1.87 (m, 2H), 2.40 (m, 1 H), 2.45 (s, 3H), 3.86 (s, 3H), 6.93 (dd, J=8.6, 2.6 Hz, 1H), 7.04 (d, J=2.6 Hz, 1 H), 7.50 (d, J=8.6 Hz, 1H), 7.50 (d, J=1.5 Hz, 1H), 7.81 (d, J=1.5 Hz, 1H).

### Example 33: 3-bromo-2-(2-chloro-4-methoxyphenyl)-6-(1-ethylpropyl)-8-methylimidazo[1,2-a]pyridine

N-bromosuccinimide (1.94 g) was added to the compound (3.55 g) obtained in Example 7(7) in acetonitrile solution (70 mL) at room temperature, followed by stirring for 30 minutes. The reaction mixture was concentrated. After added with water, the residue was extracted with ethyl acetate. An organic layer was washed with water and a saturated salt solution, and concentrated under reduced pressure after drying with anhydrous magnesium sulfate. The obtained residue was purified by silica gel chromatography (hexane:ethyl acetate=100:1→4:1), to thereby obtain a title compound (3.6 g) having the following physical properties.
TLC: Rf 0.41 (hexane:ethyl acetate=4:1)
¹H-NMR (300 Mz, CDCl₃) : δ 0.84 (t, J=7.5 Hz, 6H), 1.51-1.84 (m, 4H), 2.35 (m, 1H), 2.63 (s, 3H), 3.85 (s, 3H), 6.90 (m, 2H), 7.05 (d, J=2.4 Hz, 1H), 7.46 (d, J=8.4 Hz, 1H), 7.75 (s, 1H).

### Example 34: 2-(2-chloro-4-methoxyphenyl)-6-(1-ethylpropyl)-8-methylimidazo[1,2-a]pyridine-3-carbaldehyde

Isopropylmagnesium chloride (1.71 mL, 2.0 M ether solution) was dropped to the compound (600 mg) produced in Example 33 at 0°C, followed by stirring at room temperature for 1 hour. Dimethylformamide (300 µL) was added to the reaction mixture at 0°C, and the reaction solution was stirred at room temperature for 1 hour. After added with water, the reaction mixture was extracted with ethyl acetate. An organic layer was washed with water and a saturated salt solution to be concentrated under reduced pressure after drying with anhydrous magnesium sulfate. The obtained residue was purified by silica gel chromatography (hexane:ethyl acetate=87:13→66:34), to thereby obtain a title compound (336 mg) having the following physical properties.
TLC: Rf 0.47 (hexane:ethyl acetate=2:1);
¹H-NMR (300 MHz, CDCl₃) : δ 0.83 (t, J=7.41 Hz, 6H) 1.52-1.69 (m, 2H) 1.70-1.87 (m, 2H) 2.43 (m, 1H) 2.69 (s, 3H) 3.88 (s, 3H) 6.95 (dd, J=8.60, 2.56 Hz, 1 H) 7.07 (d, J=2.56 Hz, 1 H) 7.22 (m, 1H) 7.52 (d, J=8.42 Hz, 1H) 9.22 (m, 1H) 9.72 (s, 1H).

### Example 34(1):

The following compound was obtained by using corresponding compound and performing the same process as in Example 34.

### Example 34(1): 1-[2-(2-chloro-4-methoxyphenyl)-6-(1-ethylpropyl)-8-methylimidazo[1,2-a]pyridin-3-yl]ethanone

TLC: Rf 0.54 (hexane:ethyl acetate=2:1);
¹H-NMR (300 MHz, CDCl₃): δ 0.83 (t, J=7.3 Hz, 6H), 1.49-1.88 (m, 4H), 2.12 (s, 3H), 2.42 (m, 1H), 2.67 (s, 3H), 3.87 (s, 3H), 6.95 (dd, J=8.4, 2.6 Hz, 1H), 7.07 (d, J=2.6Hz, 1H), 7.17 (s, 1H), 7.40 (d, J=8.4 Hz, 1H), 9.44 (s, 1H).

### Example 35: [2-(2-chloro-4-methoxyphenyl)-6-(1-ethylpropyl)-8-methylimidazo[1,2-a]pyridin-3-yl]methanol

Sodium borohydride (26 mg) was added to the compound (161 mg) obtained in Example 34 in anhydrous tetrahydrofuran solution (2 mL), followed by stirring for 1 hour. Water was added to the reaction mixture, and the extract extracted with ethyl acetate was washed with water and a saturated salt solution. The resultant solution was concentrated under reduced pressure after drying with anhydrous magnesium sulfate. The obtained residue was washed with hexane/ethyl acetate, to thereby obtain a title compound (141 mg) having the following physical properties.
TLC: Rf 0.42 (hexane:ethyl acetate==1:1);
¹H-NMR (300 MHz, CDCl₃) : δ 0.83 (t, J=7.3 Hz, 6H), 1.51-1.66 (m, 2H), 1.68 - 1.81 (m, 3H), 2.32 (m, 1H), 2.62 (s, 3H), 3.85 (s, 3H), 4.86 (d, J=6.2 Hz, 2H), 6.89 - 6.93 (m, 2H), 7.03 (d, J=2.6 Hz, 1H), 7.49 (d, J=8.6 Hz, 1H), 7.93 (s, 1H).

### Example 36: 2-(2-chloro-4-methoxyphenyl)-6-(1-ethylpropyl)-8-methyl-3-(methylsulfonyl)imidazo[1,2-a]pyridine

The compound (200 mg) produced in Example 33 was dissolved in dimethylsulfoxide (1.0 mL) under argon gas atmosphere. After that, sodium methanesulfinate (58 mg), copper (I) iodide (9.0 mg), L-proline (11 mg), and sodium hydroxide (3.8 mg) were added thereto, followed by stirring at 100°C for 24 hours. After being cooled, the reaction solution was added with water and extracted with hexane and ethyl acetate (1:1). An organic layer was washed with a saturated salt solution, and dried with anhydrous magnesium sulfate, thereafter filtered with Celite, and concentrated under reduced pressure. The residue thus obtained was purified by silica gel chromatography (hexane:ethyl acetate=70:30→40:60), to thereby obtain a title compound (14.9 mg) having the following physical properties.
TLC: Rf 0.44 (hexane:ethyl acetate=1:1);
¹H-NMR (300 MHz, CDCl₃) : δ 0.85 (t, J=7.3 Hz, 6H), 1.48-1.90 (m, 4H), 2.40 (m, 1H), 2.67 (s, 3H), 3.11 (s, 3 H), 3.85 (s, 3 H), 6.91 (dd, J=8.6, 2.6 Hz, 1H), 7.03 (d, J=2.6 Hz, 1H), 7.15 (s, 1H), 7.40 (d, J=8.6 Hz, 1H), 8.51 (s, 1H).

### [Pharmacologic Experiment Example]

It was confirmed that the compound of the present invention represented by the formula (I) has a CRF receptor antagonistic activity by the following experiments.

### Experiment Example 1: Binding assay

### [Membrane Preparation]

The forced-expression cell strain of the human CRF receptor I (parent strain is CHO-K1 cell) was cultured until the cell was confluent and collected with a scraper. The collected cells were washed twice with PBS, and were suspended with an ice-colded binding assay buffer (Tris-HCl (50 mM, pH 7.0), EDTA (2 mM, pH 8.0), and MgCl₂ (10 mM)). After fractured with a dounce-type homogenizer, the suspended cells were centrifuged at 10,000 g, and a membrane fraction was collected. The collected membrane fraction was resuspended with a little amount of the binding assay buffer, thereafter diluted with the binding assay buffer so as to have a concentration of 1 mg/mL. The obtained membrane fraction was used for a binding assay.

### [Binding assay]

¹²⁵I-CRF was diluted with the binding assay buffer so as to have a concentration of 0.5 nM, whereby 50 µL of the diluted ¹²⁵I-CRF was added to a siliconized 1.5-mL tube. Next, test drugs diluted at appropriate times, such as DMSO (for general binding) or 100 µM of CRF (for non-specific binding), was added to a 1-µL tube. Finally, 50 µL of the membrane fraction was added thereto and the reaction was started (final concentration of ¹²⁵I-CRF was 0.25 nM). The tube was incubated at room temperature for 2 hours. After the reaction, the tube was centrifuged at 20,000 g in order to collect the membrane fraction. The supernatant was discarded, and the pellet was washed twice with an ice-colded PBS/0.01% TritonX-100. A membrane binding count was measured using a γ counter.

The specific binding count was obtained by subtracting the non-specific count from the measured count.

As a result, it was revealed that the compound of the present invention had a strong receptor binding activity (IC₅₀ value < 1 µM). For example, the compound of Example 7 has an IC₅₀ value of 0.012 µM.

### Experiment Example 2: Receptor antagonistic activity (cyclic AMP assay)

The forced expression cell strain of the human CRF receptor I was cultured at 37°C under 5% carbon dioxide and 95% air by using a Ham's F-12 medium (F-12 nutrient mixture) containing 10% bovine fetal serum and 1% antibiotic substance-antifungal agent. On the previous day before the cyclic AMP was measured, the cells were inoculated into a 96-well plate in an amount of 1X10⁴ cell/well. On the measurement day, the cells were washed twice with a Ham's F-12 medium, whereby a solution of Ham's F-12 medium/1 mM 3-isobutyl-1-methylxanthine (assay medium) (178 µL) was added to each well. After the incubation at 37°C for 10 minutes, test drug solutions (2 µL) in various concentrations were added thereto, or DMSO (2 µL) was added to a CRF group and a blank group. An assay medium (20 µL) containing 10 nM human/rat CRF was added to wells of a compound group and the CRF group. An assay medium (20 µL) containing 0.00001% acetic acid was added to the blank group. Each group was further incubated at 37°C for 15 minutes. The supernatant was removed and ice-colded to stop the reaction. Note that the reactions of 2 wells were the same in all reactions. The measurement for accumulated cyclic AMP amount in cells was carried out by using a cyclic AMP Biotrak enzyme immunoassay system (manufactured by Amersham Biosciences Corp.). The accumulated cyclic AMP amounts were calculated by subtracting mean values of corresponding 2 wells in the blank group from that of 2 wells. The IC₅₀ values were calculated with non-linear regression analysis by defining logarithm concentration of the compound as independent variable and an accumulated cyclic AMP amount as dependent variable.

As a result, it was revealed that the compound of the present invention has a strong antagonistic activity against CRF (IC₅₀ value < 1 µM). For example the compound of Example 7 has an IC₅₀ value of 0.041 µM.

### Experiment Example 3: Elevated plus maze test with swim-stressed rat

2 open arms with the same lengths (50 x 10 cm) and 2 close arms (a 30 cm wall was provided) with the same lengths (50 x 10 cm) were arranged in orthogonal manner at a height of 50 cm from the floor to provide an elevated plus maze system. In 70 cm above the both open arms, white light was provided, whereby illuminance was maintained at constant.

A 7-week-old SD rat (CHARLES RIVER LABORATORIES JAPAN) was forcedly made to swim for 120 seconds in a pool (40 x 30 x 38 cm) at 22°C of water temperature and 25 cm of water depth. After 9 minutes of soaking stress loading, the rat was put on the center of the system. The activity for 5 minutes of the rat was analyzed with an automatic activity-tracking analysis system (EthoVision Version 3.0, Noldus Information Technology) to calculate the dwell time (second) on the open arm.

Note that no soaking stress was loaded on the control group. In addition, 20% Solutol/HS15 was orally administered to the vehicle group and test drugs in various concentrations were orally administrated to the test drug administration group 1 hour before the test.

When the dwell time on the open arm was favorably elongated owing to the compound of the present invention compared to the dwell time on the open arm of the vehicle group, it is confirmed that the compound of the present invention has strong effects of preventing and/or treating a psychoneurotic disease, and in particular, an antianxiety effect.

### [Preparation Examples]

### Preparation Example 1:

The following components were mixed by known method, and thereafter formed into a tablet to obtain 10,000 tablets each containing 10 mg of active ingredient per tablet.

| | |
|---|---|
| 2-(2-chloro-4-methoxyphenyl)-6-(1-ethylpropyl)-3, 8-dimethylimidazo[1,2-a]pyridine | 100 g |
| Carboxymethyl cellulose calcium (disintegrator) | 20 g |
| Magnesium stearate (lubricator) | 10 g |
| Microcrystalline cellulose | 870 g |

### Preparation Example 2:

The following components were mixed by known method, followed by filtration with a dust-removing filter. The resultant filtrate in an amount of 5 mL was loaded into an ampule, and the resultant mixture was sterilized by heat with an autoclave to obtain 10,000 ampules containing 20 mg of active ingredient per ampule.

| | |
|---|---|
| 2-(2-chloro-4-methoxyphenyl)-6-(1-ethylpropyl)-3, 8-dimethylimidazo[1,2-a]pyridine | 200 g |
| Mannitol | 20 g |
| Distilled water | 50 L |

### Industrial Applicability

The compound of the present invention has a CRF antagonist action, and therefore is effective for preventing and/or treating CRF mediated diseases, for example, neuropsychiatric diseases or digestive diseases.

## Claims

1. A compound represented by formula (I), a salt thereof, an N-oxide thereof, a solvate thereof, or a prodrug thereof: 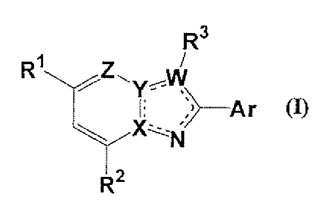 wherein X, Y, and W each independently represent a carbon atom or a nitrogen atom;
Z represents CH or a nitrogen atom;
represents a single or double bond, which does not represents a double bond successively;
R¹ represents (1) C3-10 branched alkyl which may be substituted or (2) -(CH₂)ₘ-NR⁴R⁵, in which R⁴ and R⁵ each independently represent C1-6 alkyl which may be substituted, or R⁴ represents a hydrogen atom, and R⁵ represents C3-6 branched alkyl which may be substituted, m represents 0 or an integer of 1-3;
R² and R³ each independently represent (1) a hydrogen atom, (2) C1-4 alkyl which may be substituted with a halogen atom, hydroxy which may be protected, amino which may be protected, or carboxyl which may be protected, (3) C2-4 alkenyl, (4) C2-4 alkynyl, (5) nitrile, (6) COOR⁶, (7) CONR⁷R⁸, (8) COR¹⁰¹, (9) S(O)ₙR¹⁰², or (10) a halogen atom, in which R⁶ represents a hydrogen atom or C1-4 alkyl, R⁷ and R⁸ each independently represent a hydrogen atom or C1-4 alkyl, R¹⁰¹ represents a hydrogen atom or C1-4 alkyl, R¹⁰² represents C1-4 alkyl, n represents 1 or 2; and
Ar represents an aromatic ring which may be substituted.

2. The compound according to claim 1, a salt thereof, an N-oxide thereof, a solvate thereof, or a prodrug thereof, wherein 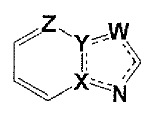 is

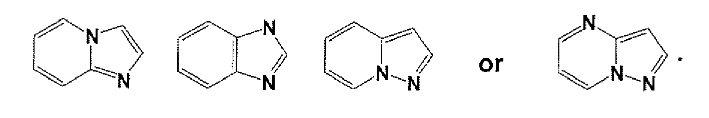

3. The compound according to claim 1, a salt thereof, an N-oxide thereof, a solvate thereof, or a prodrug thereof, wherein R¹ is C3-10 branched alkyl which may be substituted.

4. The compound according to claim 1, a salt thereof, an N-oxide thereof, a solvate thereof, or a prodrug thereof, wherein Ar has 1-3 substituent(s), and is a 5-12 membered monocyclic or bicyclic aromatic ring which may contain 1-4 hetero atoms selected from a nitrogen atom, oxygen atom and/or sulfur atom which may be oxidized.

5. The compound according to claim 4, a salt thereof, an N-oxide thereof, a solvate thereof, or a prodrug thereof, wherein Ar is a benzene having 1-3 substituent(s).

6. The compound according to claim 1, a salt thereof, an N-oxide thereof, a solvate thereof, or a prodrug thereof, wherein the formula (I) is formula (I-A-1): 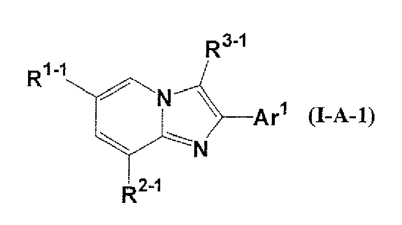 wherein R¹⁻¹ represents unsubstituted C3-10 branched alkyl,
R²⁻¹ represents a hydrogen atom, unsubstituted C1-4 alkyl or nitrile,
R³⁻¹ represents a hydrogen atom, C1-4 alkyl which may be substituted with a hydroxy which may be protected, nitrile, COOR⁶, CONR⁷R⁸, COR¹⁰¹, or S(O)ₙR¹⁰², in which all symbols represent the same meanings as described in claim 1,
Ar¹ represents a benzene having 1-3 substituent(s).

7. The compound according to claim 1, a salt thereof, an N-oxide thereof, a solvate thereof, or a prodrug thereof, wherein the formula (I) is formula (I-B-1): 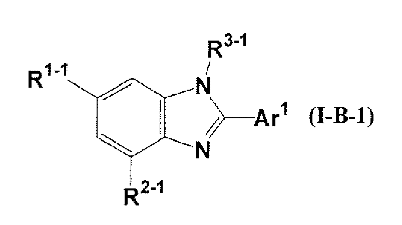 in which all symbols represent the same meanings as described in claim 6.

8. The compound according to claim 1, a salt thereof, an N-oxide thereof, a solvate thereof, or a prodrug thereof, wherein the formula (I) is formula (I-C-1): 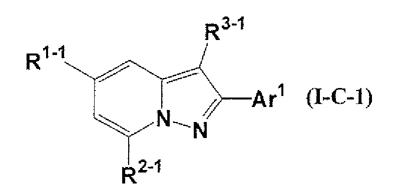 in which all symbols represent the same meanings as described in claim 6.

9. The compound according to claim 6, a salt thereof, an N-oxide thereof, a solvate thereof, or a prodrug thereof, wherein the formula (I-A-1) is formula (I-A-1-1): 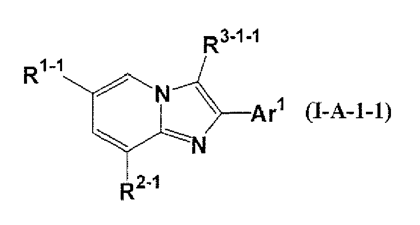 wherein R³⁻¹⁻¹ represents unsubstituted C1-4 alkyl, or CONR⁷R⁸, in which all symbols represent the same meanings as described in claim 1 and the other symbols represent the same meaning as described in claim 6.

10. The compound according to claim 1, a salt thereof, an N-oxide thereof, a solvate thereof, or a prodrug thereof, wherein the compound represented by the formula (I) is
(1) 2-(2-chloro-4-methoxyphenyl)-6-(1-ethylpropyl)-3,8-dimethylimidazo[1,2-a]pyridine,
(2) 3-ethyl-6-(1-ethylpropyl)-2-(4-methoxy-2-methylphenyl)-8-methylimidazo[1,2-a]pyridine,
(3) 2-(2-chloro-4-methoxyphenyl)-3-ethyl-6-(1-ethylpropyl)-8-methylimidazo[1,2-a]pyridine,
(4) ethyl 2-(2-chloro-4-methoxyphenyl)-6-(1-ethylpropyl)-8-methylimidazo[1,2-a]pyridine-3-carboxylate,
(5) methyl 2-(2-chloro-4-methoxyphenyl)-6-(1-ethylpropyl)-8-methylimidazo[1,2-a]pyridine-3-carboxylate,
(6) 2-(2-chloro-4-methoxyphenyl)-6-(1-ethylpropyl)-8-methylimidazo[1,2-a]pyridine-3-carboxamide,
(7) 2-(2-chloro-4-methoxyphenyl)-6-(1-ethylpropyl)-N,8-dimethylimidazo[1,2-a]pyridine-3-carboxamide,
(8) 2-(2-chloro-4-methoxyphenyl)-6-(1-ethylpropyl)-1,4-dimethyl-1H-benzimidazole, or
(9)2-(2-chloro-4-methoxyphenyl)-5-(1-ethylpropyl)-7-methylpyrazolo[1,5-a]pyridine-3-carboxamide.

11. The compound according to claim 6, a salt thereof, an N-oxide thereof, a solvate thereof, or a prodrug thereof, wherein the compound represented by the formula (I-A-1) is
(1) 6-(1-ethylpropyl)-2-(4-methoxy-2-methylphenyl)-8-methylimidazo[1,2-a]pyridine-3-carboxamide,
(2)6-(1-ethylpropyl)-8-methyl-2-(2,4,5-trimethylphenyl)imidazo[1,2-a]pyridine-3-carboxamide,
(3) 2-(2-ethyl-4-methoxyphenyl)-6-(1-ethylpropyl)-8-methylimidazo[1,2-a]pyridine-3-carboxamide,
(4) 2-(4-ethoxy-2-ethylphenyl)-6-(1-ethylpropyl)-8-methylimidazo[1,2-a]pyridine-3-carboxamide,
(5) 2-(2-chloro-4-methoxy-5-methylphenyl)-6-(1-ethylpropyl)-8-methylimidazo[1,2-a]pyridine-3-carboxamide,
(6) 1-[2-(2-chloro-4-methoxyphenyl)-6-(1-ethylpropyl)-8-methylimidazo[1,2-a]pyridin-3-yl]ethanone,
(7) 2-(4-ethoxy-2-methylphenyl)-6-(1-ethylpropyl)-8-methylimidazo[1,2-a]pyridine-3-carboxamide, or
(8) 6-(1-ethylpropyl)-2-(4-methoxy-2,5-dimethylphenyl)-8-methylimidazo[1,2-a]pyridine-3-carboxamide.

12. A pharmaceutical composition comprising as an active ingredient the compound represented by the formula (I) described in claim 1, a salt thereof, an N-oxide thereof, a solvate thereof, or a prodrug thereof.

13. The pharmaceutical composition according to claim 12, which is a CRF antagonist.

14. The pharmaceutical composition according to claim 13, which is an agent for preventing and/or treating CRF mediated diseases.

15. The pharmaceutical composition according to claim 14, wherein the CRF mediated diseases are neuropsychiatric diseases or digestive diseases.

16. The pharmaceutical composition according to claim 15, wherein the neuropsychiatric diseases or the digestive diseases are mood disorder, anxiety disorder, adjustment disorder, stress-related disorder, eating disorder, symptom caused by psychotropic substance or dependency thereon, organic mental disorder, schizophrenic disorder, attention-deficit hyperactivity disorder, irritable bowel syndrome, or gastrointestinal disorder caused by stress.

17. The pharmaceutical composition according to claim 16, wherein the mood disorders are depression, bipolar disorder, indefinite complaint, premenstrual dysphoric disorder, postpartum mood disorder, or perimenopausal or menopausal dysphoric disorder, and the anxiety disorders are generalized anxiety disorder, panic disorder, obsessive compulsive disorder, social anxiety disorder, or phobic disorder.

18. A pharmaceutical composition comprising the compound represented by the formula (I) described in claim 1, a salt thereof, an N-oxide thereof, a solvate thereof, or a prodrug thereof in combination with at least one kind selected from a tricyclic antidepressant, a tetracyclic antidepressant, a monoamine oxidase inhibitor, a serotonin and noradrenaline reuptake inhibitor, a selective serotonin reuptake inhibitor, a serotonin reuptake inhibitor, a psychostimulant, an antianxiety agent, an antipsychotic agent, a mitochondrial benzodiazepine receptor ligand, a neurokinin 1 antagonist, a gastrointestinal promotility agent, a histamine H₂ receptor antagonist, a proton pump inhibitor, a 5-HT₃ antagonist, a 5-HT₄ agonist, an anticholinergic agent, an antidiarrheal drug, a laxative, and an autonomic modulating agent.

19. A method of preventing and/or treating CRF mediated diseases, comprising administering to a mammal an effective amount of the compound represented by the formula (I) described in claim 1, a salt thereof, an N-oxide thereof, a solvate thereof, or a prodrug thereof.

20. A use of the compound represented by the formula (I) described in claim 1, a salt thereof, an N-oxide thereof, a solvate thereof, or a prodrug thereof for manufacturing an agent for preventing and/or treating CRF mediated diseases.
